# EUROPEAN PATENT APPLICATION

(11) **EP 4 238 581 A1**
(43) Date of publication of application: **06.09.2023**
(21) Application number: 22160227.9
(22) Date of filing: 04.03.2022
(51) Int. Cl.: A61K 47/59, A61K 47/68, A61K 47/64, A61K 47/54, A61K 47/61, A61K 45/06, C07K 16/28, C07K 16/30, C07K 14/195, C12N 15/87, A61P 3/06, A61P 31/12, A61P 35/00

(54) **TARGETED DELIVERY OF OLIGONUCLEOTIDES INTO EUKARYOTIC CELLS USING HYBRID MALTOSE/CYCLODEXTRIN POLYPLEXES**

(71) Applicant: Technische Universität Dresden e. V., 01069 Dresden (DE); Leibniz-Institut für Polymerforschung e. V., 01069 Dresden (DE)
(72) Inventor: TEMME, Achim, 01069 Dresden (DE); JUGEL, Willi, 01069 Dresden (DE); TIETZE, Stefanie, 01069 Dresden (DE); APPELHANS, Dietmar, 01069 Dresden (DE)
(74) Representative: Maikowski & Ninnemann Patentanwälte Partnerschaft mbB

(57) **Abstract**

The present invention relates generally to the field of molecular biology and therapeutics. More specifically, the present invention relates to a novel targeting bio-conjugate for selective delivery of therapeutic nucleic acids, including RNA oligonucleotides and DNA-oligonucleotides, preferably gene-encoding DNA plasmids or minicircle DNAs, to eukaryotic cells by means of receptor-mediated endocytosis and endosomal release. The invention is generally related to a system for targeted delivery of nucleic acid molecules, comprising a core, which consists of avidin or neutravidin, at least one antibody, which is preferably a biotinylated antibody, which is preferably an antibody single-chain variable fragment, conjugated to hybrid polyplexes comprising maltose and cyclodextrin molecules. The invention further provides a method for the assembly of said delivery system and the use of said delivery systems in the therapy of metabolic diseases, such as familial hypercholesterolemia, viral infections, and proliferative diseases, such as primary tumors or metastatic cancers.

## Description

### Field of the invention

The present invention relates generally to the field of molecular biology and therapeutics. More specifically, the present invention relates to a novel targeting bio-conjugate for selective delivery of therapeutic nucleic acids, including RNA oligonucleotides and DNA-oligonucleotides, preferably gene-encoding DNA plasmids or minicircle DNAs, to eukaryotic cells by means of receptor-mediated endocytosis and endosomal release. The invention is generally related to a system for targeted delivery of nucleic acid molecules, comprising a core, which consists of avidin or neutravidin, at least one antibody, which is preferably a biotinylated antibody, which is preferably an antibody single-chain variable fragment, conjugated to hybrid polyplexes comprising maltose and cyclodextrin molecules. The invention further provides a method for the assembly of said delivery system and the use of said delivery systems in the therapy of metabolic diseases, such as familial hypercholesterolemia, viral infections, and proliferative diseases, such as primary tumors or metastatic cancers.

### Description of related art

Efficient and non-toxic DNA delivery is still a major limiting factor for non-viral gene therapy. Among the large variety of non-viral carrier systems, the cationic polymers, such as poly(ethylene imine) (PEI), poly(propylene imine) (PPI) and poly(amido amine) (PAMAM) play a prominent role in nucleic acid delivery. However, limitations of polycationic polymer-based DNA delivery systems, especially when applying in vivo are (i) cytotoxicity, (ii) insufficient target specificity, (iii) unsatisfactory transgene expression and (iv) undesired transfer of therapeutic DNA into non-target cells and organs such as lung and liver.

One concept to overcome the limitations is called targeted delivery, which uses the receptors and endocytosis machinery of cells to deliver polymeric carriers and their oligonucleotide payload. Essential prerequisites for enabling this receptor-mediated endocytosis are (i) arming of the polymeric carriers with targeting devices, preferably antibodies, and (ii) decreasing cationic surface charge of polycationic carriers. The latter avoids electrostatic interactions with negatively charged structures of the cell membrane, such as sialo-glycoproteins and lipid-bound sialo acids, leading to unspecific uptake.

Such a delivery system, designated polyplex, preferably comprising maltose-modified PPI and biotinylated PPI carrier molecules, neutravidin, mono-biotinylated single chain antibodies was competent to efficiently deliver siRNA molecules to tumor cells expressing the cognate receptor (Tietze et al., 2017).

However, delivery of high molecular weight oligonucleotides such as DNA plasmids and mincircle DNA using this system, showed selective delivery but insufficient gene transfer. It was concluded that sugar modification of cationic PPI carriers negatively affects endosomal release and DNA transfer to the cytosol.

The prior art is deficient in the absence of an selective carrier system for DNA, exclusively assembled by means of biotin-avidin interactions on a modular basis, which upon endocytosis into eukaryotic cells sufficiently escape the endosomal compartment and therefore enables efficient transgene expression in the targeted cell. The present invention fulfills this longstanding need and desire in the art.

### Background of the invention

Gene therapy for the treatment of malignancies remains an area of particular interest. Electroporation of DNA or mRNA is previously employed for gene engineering of eukaryotic cells. Yet, this delivery approach is generally not applicable *in vivo.* Furthermore, the major disadvantage of electroporation is substantial cell death caused by high voltage pulses and only partially successful membrane repair eventually resulting in cell death and therefore requiring the use of greater quantities of cells compared to chemical transfection methods (Hui, 2008). Previous attempts to deliver gene-coding DNA using replication-deficient viral vectors showed only limited success in preclinical and clinical studies, and have also raised serious safety concerns (Check, 2002; Thomas et al., 2003). In comparison, non-viral delivery systems tend to be simple to manufacture, show lower immunogenicity, and are associated with fewer regulatory issues when translated into clinical settings. However, the broad application of therapeutic DNA using non-viral delivery systems such as liposomes, cationic lipids, inorganic particles, polymers and combinations thereof, is hampered by non-specific toxic side effects, poor pharmacokinetics, low DNA transfection efficiency and gene expression duration (for review see (Al-Dosari and Gao, 2009)).

One approach to avoid unwanted off-target effects of non-viral vectors delivering nucleic acids is the introduction of targeting devices, such as antibodies and ligands for cellular surface receptors that specifically bind to target cells, leading to the concept of targeted transfection (referred as to targeted delivery). However, this requires the identification of optimal targeting devices, their coupling to a nucleic acid-carrier complex in a way that retains their binding activity, and the further modifications that avoid non-specific uptake by non-target cells, in particular by cells of the reticuloendothelial system (i.e. mononuclear phagocytic system) and scavenger endothelial cells (SEC) (Hayashi et al., 2020).

Furthermore, it is unanimously recognized, that for proper evaluation of targeted transfection it is mandatory to use a stringent read out system to exclude any bias which can be caused by residual expression levels of the targeted cellular receptor on negative controls, by the composition of cell membranes (i.e. different cell surface charge on cell lines) or functional dissimilarities in their endocytic machinery. For this purpose target cells with expression of the targeted surface antigen and isogenic controls must be included. Optionally, an irrelevant but structurally similar targeting device can be employed in the non-viral vector systems to accurately evaluate targeted delivery and transfection efficacy.

So far, only a limited number of results for targeted non-viral delivery of gene encoding nucleic acids according to the above-mentioned prerequisites were described. For instance, anti-E-selectin-receptor monoclonal antibody-modified liposomes loaded with plasmid-DNA encoding β-Galactosidase shows a significant approximately threefold better transgene expression levels into CHO cells genetically engineered to express E-selectin (CHO-E) when compared to liposomes devoid of antibodies or coupled to a control antibody. Likewise, an approximately 3-fold improved transgene expression was revealed in CHO-E cells when compared to CHO wild type cells or CHO engineered to express CTLA4 Ig, respectively (Tan et al., 2003). Yet, caution is advised when choosing β-galactosidase as surrogate marker for DNA-transfer, since higher endogenous lysosomal beta-galactosidase activity in senescent and stressed cells eventually produce false positive results (Alessio et al., 2021). A similar report from the same group reports transfection of EGFP plasmids with PAMAM dendrimers conjugated with anti-E-selectin antibody resulting in a significant approximately 2.7-fold increased gene transfer in CHO-E cells when compared to PAMAM dendrimers conjugated with a non-binding control antibody or PAMAM dendrimers only loaded with the EGFP-encoding plasmid. Yet, by using this E-Selectin-selective system transfection efficiency was reported below 30% for E-selectin expressing cells (Theoharis et al., 2009).

An alternative way to modify non-viral particles for targeted transfection, is the use of single chain antibodies fused to anionic peptides, designated immunoporter. Conceptually, the anionic peptide tail of single chain antibodies allow electrostatic interaction with polycationic carriers complexed with DNA, while maintaining receptor specificity. Yet, a recent report targeting EGFR on various tumor cells described only minimal gene transfer in the range of 3% - 5%. In this study, the reported increase in sensitivity of EGFR-positive A431 tumor cells transfected with the suicide gene Herpes Virus Thymidine Kinase (HSV-TK) towards the virostatic drug ganciclovir was most likely due to intercellular diffusion of the processed ganciclovir nucleoside through gap junctions and therefore does not reflect transfection efficiency (Suzuki et al., 2003).

Similar to the above mentioned approach, T cell-targeting nanoparticles based on poly(beta-amino-ester) (PBAE) polymers were generated by assembly with anti-CD3e f(ab)2 fragments chemically modified with anionic poly-glutamatic acid. In order to improve transfection efficacy a peptide containing a nuclear localization signal was included. Yet, *in vitro* transfection efficiency of such nanoparticles delivering a plasmid encoding an anti-CD 19 chimeric antigen receptor and a genetically linked EGFP reporter gene into primary murine T were low. More specifically, transfection with a ratio of 3,000 nanoparticles per T cell resulted 30 hours after treatment in the mean in only 3.8% EGFP-positive T cells (Smith et al., 2017). An alternative method for targeted delivery of DNA, and negatively charged oligonucleotides in general, comprises the cationic protein protamine (an oligonucleotide carrier molecule derived from sperm of fish), fused or chemically coupled to cell surface receptors-internalizing antibodies. Conceptually, cationic peptide tails of single chain antibodies allow electrostatic interaction with the negatively charged phosphoribose and phosphodesoxyribose backbones of RNA and DNA, respectively. In a recent report, anti-human epidermal growth factor receptor 2 (ErbB2/EGFR-2) antibody fused with a truncated protamine proportion was used to deliver luciferase reporter gene into difficult to transfect ErbB2-positive breast cancer cell line SKBR3. Although, some Luc-transgene activity was detected, enzymatic activity was ten-fold lower compared to the luciferase activities observed when using the suboptimal potassium-phosphate transfection protocol. Furthermore, no selective gene transfer was noted when MCF7 cells, which are well known for ErbB2 surface expression, were employed (Li et al., 2001). This observed inefficient transgene delivery prompted the authors to investigate a modified delivery system comprising cationic DOTAP lipid, protamine, unmodified anti-erbB2 scFv and a cLuc-encoding plasmid, which was proposed to assembly exclusively by electrostatic interaction. To this end, by using this modified system, gene delivery remained ineffective. In addition, it is well known that serum components inhibits DOTAP-mediated gene transfer, which render DOTAP or derivatives thereof not suitable for *in vivo* applications.

As mentioned above, protamine can be chemically coupled to targeting devices. A well-known method is the modification of protamine with the bispecific cross-linker sulfo-SMCC (sulfosuccinimidyl 4-(N-maleimidomethyl)cyclohexane-1-carboxylate) and the subsequent chemical coupling to cysteine residues of antibodies (Bäumer et al., 2016). Employing this chemical coupling can cause unwanted chemical reactions leading to adverse effects in the binding properties of the antibodies, and therefore must be tested which each individual antibody. Noteworthy, protamine alone possesses intrinsic capacity to cross cellular membranes by unspecific endocytic uptake (Dinauer et al., 2004; Pardridge et al., 1993) and therefore off-target effects of antibody-protamine nucleic acid carriers cannot completely be ruled out. Among cationic polymer gene transfer systems, poly(propylene imine) (PPI) and its surface-modified derivatives have emerged as promising DNA carriers. The high rate of positively charged amino groups on the surface of the PPI dendrimers enables electrostatic interaction with the negatively charged DNA (Dufès et al., 2005) and result in the formation of compact nano-sized particles, designated "dendriplexes" (Shcharbin et al., 2009). Tuning PPI by surface modifications with for instance poly(ethylene glycol) (PEG) reduces cytotoxicity and inhibits intermolecular aggregation. In addition, surface modifications provide a hydrophilic shell that avoids interaction with the reticuloendothelial system and scavenger endothelial cells and prolongs circulation time in the bloodstream (Lee et al., 2001). Recently, it has been shown that surface charge shielding by maltose modification of peripheral amino groups greatly improves the biocompatibility of PPI glycodendrimers (mal19-PPI) while simultaneously reducing transfection efficiency. However, upon bioconjugation of mono-biotinylated single chain antibodies (scFv-P-BAP) for targeting, PPI glycodendrimers as so-called "polyplexes" became competent for delivering siRNA to target cells *in vitro* and *in vivo* expressing the cognate antigen/receptor (Jugel et al., 2021; Tietze et al., 2017).

However, delivery of high molecular weight oligonucleotides such as DNA-plasmids and mincircle DNA using this system, showed selective delivery but insufficient transfection efficiency below 35% when targeting 293T cells expressing a cognate receptor. It was concluded that the modification of cationic PPI carriers negatively affects endosomal release and DNA transfer to the cytosol.

In order to improve endosomal escape of polyplexes and gene encoding DNA, a novel hybrid polyplex system combining transfection-incompetent antibody-conjugated mal19-PPIs with transfection-competent β-cyclodextrin-modified PPIs (CD-PPI) has been developed by the inventors.

Such β-cyclodextrin-containing polyplexes show a shift towards neutral net charge when conjugated to neutravidin and scFv-BAP, and were unexpectedly highly efficient to selectively transfect target cells with expression of the cognate receptor.

### Description of the invention

### Definitions

As used herein, the expressions "cell", "cell line," and "cell culture" are used interchangeably and all such designations include progeny. Thus, the words "transformants" and "transformed cells" include the primary subject cell and culture derived therefrom without regard for the number of transfers. It is also understood that all progeny may not be precisely identical in DNA content, due to deliberate or inadvertent mutations. Mutant progeny that have the same function or biological activity as screened for in the originally transformed cell are included. Where distinct designations are intended, this will be clear from the context.

The terms "polypeptide", "peptide", and "protein", as used herein, are interchangeable and are defined to mean a biomolecule composed of amino acids linked by a peptide bond.

If peptide or amino acid sequences are mentioned herein, each amino acid residue is represented by a one-letter or a three-letter designation, corresponding to the trivial name of the amino acid, in accordance with the following conventional list:

| Amino Acid | One-Letter Symbol | Three-Letter Symbol |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

The terms "a", "an" and "the" as used herein are defined to mean "one or more" and include the plural unless the context is inappropriate.

The term "subject" as used herein, refers to an animal, preferably a mammal, most preferably a human, who has been the object of treatment, observation or experiment.

The term "therapeutically effective amount" as used herein, means that amount of active compound or pharmaceutical agent that elicits the biological or medicinal response in a tissue system, animal or human being sought by a researcher, veterinarian, medical doctor or other clinician, which includes alleviation of the symptoms of the disease or disorder being treated.

As used herein, the term "pharmaceutically acceptable" embraces both human and veterinary use: For example the term "pharmaceutically acceptable" embraces a veterinarily acceptable compound or a compound acceptable in human medicine and health care.

A single-chain variable fragment (scFv) is not actually a fragment of an antibody, but instead is a fusion protein of the variable regions of the heavy (VH) and light chains (VL) of immunoglobulins, connected with a short linker peptide of ten to about 25 amino acids. The linker is usually rich in glycine for flexibility, as well as serine or threonine for solubility, and can either connect the N-terminus of the VH with the C-terminus of the VL, or vice versa. This protein retains the specificity of the original immunoglobulin, despite removal of the constant regions and the introduction of the linker. Divalent (or bivalent) single-chain variable fragments (di-scFvs, bi-scFvs) can be engineered by linking two scFvs. This can be done by producing a single peptide chain with two VH and two VL regions, yielding tandem scFvs. For a review of scFv see (Plückthun, 1994).

### Detailed description of the invention

Non-viral transfection of nucleic acids, using different delivery systems or carriers described above, is hampered by unspecific toxic or immunogenic side effects, poor pharmacokinetics due to unwanted delivery into non-target organs or rapid renal excretion, as well as inefficient internalization into target.

In one aspect WO 2018/078076 A1 teaches a polyplex system for selective delivery of nucleic acid payload, preferably maltose-PPI-complexed siRNA exclusively assembled by biotin-neutravidin conjugation to a tetrameric biotin-binding element and mono-biotinylated cell binding component, preferably mono-biotinylated single-chain variable fragments (scFv). This polyplex system can also be used for selective transfection of DNA. Yet, insufficient endosomal escape accompanied by only moderate gene transfer and expression was noted when using such maltose-PPI-based polyplexes. It is therefore a particular purpose of the present invention to provide a delivery system for targeted delivery of gene-encoding nucleic acids, wherein said delivery system is able to overcome the disadvantages of the prior art. The invention shall enable the stable coupling of antibodies, antibody derivatives and ligands with carrier molecules electrostatically complexed with gene-encoding nucleic acids. For this purpose, mono-biotinylated antibody single-chain variable fragments (scFv) are conjugated to nucleic acid/polymer nanoparticles via tetrameric biotin-binding proteins, such as avidin, neutravidin, or streptavidin.

The present invention provides the modular design and assembly of a hybrid polyplex system exclusively assembled by means of biotin-binding element conjugation, comprising maltose and cyclodextrin-modified PPI dendritic polymers, mono-biotinylated cell binding component, a tetrameric biotin-binding element, and nucleic acid payload, preferably plasmid DNA and minicircle DNA, for therapeutic and diagnostic purposes. The hybrid polyplex system provides selective transfection of the nucleic acid payload into cells expressing the cognate surface receptor.

Further possible therapeutic or diagnostic agents, which can be used as payload by the invention, are selected from RNA and DNA molecules. Polymers or cationic charged carrier systems for therapeutic RNA or DNA molecules have already been described. However, these carrier systems lead to a non-specific uptake in both tumor cells as well as in healthy body cells. The use of active compounds or the carrier systems can thus lead to the damage of normal cells or tissues (off-target effects). The targeted nucleic delivery system of the invention enable endosomal escape and subsequently the selective expression of RNA and DNA molecules in cells, which express a specific surface protein, but prevent the non-specific uptake into cells without expression of this surface protein.

By conjugation to a mono-biotinylated targeting device, i.e. mono-biotinylated scFvs, the invention can also be used to transport other therapeutically or diagnostically relevant nucleic acids specifically into eukaryotic cells, which express a specific surface antigen.

In a preferred embodiment, the invention provides a delivery system as described herein, wherein said delivery system binds, through at least one antibody single-chain variable fragment, to a surface antigen, which is specifically expressed at or in cell membranes of cancer cells.

Examples of therapeutically or diagnostically active ingredients are
- nucleic acid payloads selected from :
   - "small interfering RNA" (siRNA),
   - microRNA (miRNA)
   - non-coding RNA (ncRNA)
   - double-stranded (ds)RNA
   - CpG oligodeoxynucleotides (CpG-ODNs)
   - plasmid DNA
   - minicircle DNA
- and
   ∘ ribozymes
   wherein each ingredient is complexed with a biotinylated hybrid polyplex comprising maltose-PPI and cyclodextrin-PPI, wherein PPI is defined as poly(propylene imine) (PPI).

Most preferred according to the invention is plasmid DNA and/or minicircle DNA. Plasmid DNA or DNA plasmids are known to the person skilled in the art. DNA minicircles are small (~4kb) circular plasmid derivatives that have been freed from all prokaryotic vector parts. They have been applied as transgene carriers for the genetic modification of mammalian cells, with the advantage that, since they contain no bacterial DNA sequences characterized by non-methylated CpG motifs , they are less likely to be perceived as pathogen-associated molecular pattern (PAMP). Consequently, the use of minicircle DNA limits potential activation of pattern recognition receptors such as TLR9 and therefore avoid unwanted inflammatory responses interfering with transgene delivery and transgene expression. (Typical transgene delivery methods involve plasmids, which contain foreign DNA.) The smaller size of minicircles also improves the packaging in polycationic carriers and delivery into cells.

The present invention provides a delivery system according to claim 1. More specifically, the present invention provides a modular delivery system for delivery of nucleic acids payloads into the cytosol of a cell and targeted transfection, comprising
- an avidin tetrameric biotin-binding core (avidin core),
- at least one targeting molecule such as a natural or artificial protein-ligand, antibody, aptamer or antibody single-chain variable fragment,
- at least one nucleic acid payload selected from DNA and RNA,
wherein said nucleic acid payload is complexed with a biotinylated hybrid polyplex comprising maltose-PPI dendrimers and cyclodextrin-PPI dendrimers, wherein PPI is defined as poly(propylene imine) (PPI) and wherein said at least one targeting molecule and said biotinylated hybrid polyplex are conjugated to said avidin tetrameric biotin-binding core.

The avidin tetrameric biotin-binding core consists of a molecule selected from the group consisting of avidin, neutravidin or streptavidin.

Avidin is a tetrameric biotin-binding protein produced in the oviducts of birds, reptiles and amphibians and deposited in the whites of their eggs. The tetrameric protein contains four identical subunits (homotetramer), each of which can bind to biotin (vitamin B7, vitamin H) with a high degree of affinity and specificity. The dissociation constant of avidin is measured to be K_{D} ≈ 10-15 ^{M}, making it one of the strongest known non-covalent bonds. In its tetrameric form, avidin is estimated to be between 66-69 kDa in size. 10% of the molecular weight is attributed to carbohydrate content composed of four to five mannose and three N-acetylglucosamine residues. The carbohydrate moieties of avidin contain at least three unique oligosaccharide structural types that are similar in structure and composition.

Streptavidin is a loosely related protein with equal biotin affinity and a very similar binding site and is made by certain strains of bacteria of *Streptomyces spec..* Streptavidin is thought to serve to inhibit the growth of competing bacteria, in the manner of an antibiotic.

A non-glycosylated form of avidin is available and is known as so-called neutravidin.

The avidin core suitably consists of avidin, its none-glycosylated form neutravidin or streptavidin. More preferably, the avidin core consists of avidin or neutravidin. Most preferably, the avidin core of the delivery system of the invention consists of one molecule avidin or one molecule neutravidin.

The avidin, neutravidin or streptavidin molecule, which forms the core of the delivery system of the invention, can bind up to four biotin molecules or other molecules each of them displaying one biotin.

In one embodiment, the delivery system of the invention comprises at least one targeting molecule, such as a single-chain variable fragment of an antibody (scFv), preferably one, two or three targeting molecules, such as single-chain variable fragments, which are each fused to a biotinylation acceptor peptide (BAP).

These targeting molecules, specifically these antibody single-chain variable fragments (scFv) are responsible for binding to a cell surface receptor protein, which is expressed specifically by the selected cell type or cancer cell targets. Such cell surface receptor proteins are for example the cancer-associated Prostate Stem Cell Antigen (PSCA) or any other suitable cell surface protein or peptide, which is suitable to fulfill the purpose of the invention. In other words, the choice of the antibody single-chain variable fragment determines the specificity of the delivery system of the invention for specific cancers. Preferred according to the invention are single-chain variable fragments that bind to PSCA.

The delivery system of the invention preferably contains at least two, most preferably two antibody single-chain variable fragments (scFv), which represent at least two antigen-binding sites. These at least two antigen-binding sites are implemented in order to induce "clustering effects" and endocytosis by crosslinking of at least two receptors, such as PSCA on the surface of the targeted cell for improved cellular internalization.

In a preferred embodiment, the single chain antibodies used in the delivery system according to the invention are selected from hybridoma 7F5-derived monoclonal antibody (Morgenroth et al., 2007). In a more preferred embodiment, the single chain antibodies used in the delivery system according to the invention are selected from scFv(AM1) (SEQ ID NO: 1), scFv(h-AM-1) (SEQ ID NO: 12) and scFv(MR1.1) (SEQ ID NO: 13). Most preferably, the single chain antibody used in the delivery system according to the invention is scFv(AM1) (SEQ ID NO: 1) or scFv(h-AM-1) (SEQ ID NO: 12).

The binding between biotin and streptavidin or avidin is one of the strongest known non-covalent biological interactions. The (strept)avidin-biotin interaction has been widely used for decades in biological research and biotechnology. Therefore labeling of purified proteins by biotin is a powerful way to achieve protein capture, immobilization, and functionalization, as well as multimerizing or bridging molecules. Chemical biotinylation often generates heterogeneous products, which may have impaired function. Thus, enzymatic biotinylation, for example with *E. coli* biotin ligase (BirA) is highly specific in covalently attaching biotin to a BAP, giving a homogeneous product with high yield. A BAP can conveniently be added genetically at the N-terminus, C-terminus or in exposed loops of a target protein. Preferred according to invention is the addition of the BAP at the C-terminus of the antibody single-chain variable fragment.

Preferred BAPs according to the invention are selected from protein domains and peptides that are suitable for enzymatic biotinylation with *E. coli* biotin ligase (BirA). One suitable amino acid sequence for biotinylation comprises the biotin-accepting domain of the 1.3S subunit of *Propionibacterium shermanii* transcarboxylase (PSTCD-BAP) (MKLKVTVNGTAYDVDVDVDKSHENPMGTILFGGGTGGAPAPAAGGAGAGKAGEG EIPAPLAGTVSKILVKEGDTVKAGQTVLVLEAMKMETEINAPTDGKVEKVLVKERDA VQGGQGLIKIGDLEL SEQ ID NO. 2) as well as of the biotinyl-domain or biotin carboxyl carrier protein (BCCP) domain present in all biotin-dependent enzymes, such as acetyl-CoA carboxylase, pyruvate carboxylase, propionyl-CoA carboxylase, methylcrotonyl-CoA carboxylase, geranyl-CoA carboxylase, oxaloacetate decarboxylase, methylmalonyl-CoA decarboxylase, transcarboxylase and urea amidolyase; and present in the "cd06850" sequence cluster (http://www.ncbi.nlm.nih.gov/Structure/cdd/cddsrv.cgi?uid=cd06850) (VLRSPMPGVVVAVSVKPGDAVAEGQEICVIEAMKMQNSMTAGKTGTVKSVHCQA GDTVGEGDLLVELE, SEQ ID NO: 3) A suitable BAP peptide is for example a 13 amino acid peptide, which comprises the minimal substrate peptide for BirA:
LX₁X₂IFEAQKIEWR (SEQ ID NO: 4), wherein
X₁ = any amino acid; and
X₂ = is any amino acid except L, V, I, W, F or Y

More preferably, a suitable BAP comprises an amino acid sequence that has been further optimized to improve the rate of biotinylation, resulting in BAP called AviTag and having the amino acid sequence GLNDIFEAQKIEWHE (SEQ ID NO: 5). AviTag works at either the N or C terminus of the target protein. Further preferably, the BAP may be close 15 residue relative, termed BioTag (ALNDIFEAQKIEWHA, SEQ ID NO: 6). Another suitable BAP, BLRP (Biotin ligase recognition peptide) contains a core of AviTag and consists of 23 amino acid residues: (MAGGLNDIFEAQKIEWHEDTGGS, SEQ ID NO: 7). Another suitable BAP termed Bio-Tag also contains the core of AviTag and consists of 23 amino acids: (MSGLNDIFEAQKIEWHEGAPSSR, SEQ ID No: 8 [66]. Another suitable BAP is the 15 amino acid residue "BirA Substrate Peptide" (BSP), having the amino acid sequence LHHILDAQKMVWNHR (SEQ ID NO: 9).

In a further embodiment, a linker peptide is added between the antibody single-chain variable fragment and the BAP in order to add some flexibility between the BAP and the antibody single-chain variable fragment. For example, a flexible two amino acid residue GS linker can be added between the BAP and the antibody single-chain variable fragment or any other surrounding peptide tag or domain. In the unlikely event that constructs with N-terminal or C-terminal BAP do not enable biotinylation or yield low amounts of protein, the linker peptide can be extended to up to 6 amino residues. A preferred linker peptide according to invention is a linker peptide comprising, consisting essentially of or consisting of a c-myc tag. A c-myc tag is a polypeptide protein tag derived from the c-myc gene product that can be added to a protein using recombinant DNA technology. Most preferably, said c-myc tag has the amino acid sequence of EQKLISEEDL (SEQ ID NO: 10).

In a further preferred embodiment, the targeting molecule used in the target delivery system of the invention can also be an aptamer. Aptamers (are oligonucleotide or peptide molecules that bind to a specific target molecule. Aptamers are usually created by selecting them from a large random sequence pool, but natural aptamers also exist in riboswitches. Aptamers can be used for both basic research and clinical purposes as macromolecular drugs. Aptamers can be combined with ribozymes to self-cleave in the presence of their target molecule. These compound molecules have additional research, industrial and clinical applications. More specifically, aptamers can be classified as:
- DNA or RNA aptamers. They consist of (usually short) strands of oligonucleotides.
- peptide aptamers. They consist of one (or more) short variable peptide domains, attached at both ends to a protein scaffold.

The therapeutically active nucleic acid, which is comprised in the delivery system according to invention may be a gene-encoding plasmid or derivatives thererof such as minicircle DNA and DNA comprising genetic elements for transposition such as transposons, gene-encoding single strand RNA (ssRNA) such as in vitro-transcribed mRNA or a double strand RNA (dsRNA), of the latter with a length exceeding 40 bp, respectively. Preferably, the therapeutically active nucleic acid, which is comprised as payload in the delivery system according to invention, is selected from gene-encoding plasmid DNA and minicircle DNA.

The hybrid polyplexes comprise maltose-PPI dendrimers and cyclodextrin-PPI dendrimers, wherein PPI is defined as poly(propylene imine) (PPI).

The maltose-PPI dendrimers are suitably mal19-PPI dendrimers. These are maltose-(mal)-modified fourth-generation PPI dendrimers (mal-PPI) with 19% (mal19-PPI) being modified (14,900 g/mol; modified with 24 maltose units per molecule). Said mal19-PPI dendrimers, in which the surface charge shielding by maltose modification of peripheral amino groups greatly improves the biocompatibility of PPI glycodendrimers while simultaneously reducing transfection efficiency.

To improve DNA transfection efficiency of the polyplexes comprised in the delivery system of the invention, said polyplexes are modified, designated as hybrid poylplexes, combining transfection-incompetent antibody-conjugated mono-biotinylated mal19-PPI dendrimers (15,490 g/mol) with β-cyclodextrin-modified PPI dendrimers. β-cyclodextrins are natural cyclic oligosaccharides with 7 glucose units in their structure linked by α-(1,4) glucoside bonds. The donut-shaped β-cyclodextrins are characterized by a hydrophilic outer surface coated with hydroxyl groups and a hydrophobic inner cavity coated with ether groups of anomeric oxygen atoms. Noteworthy, β-cyclodextrins are approved by the FDA. Preferably, β-cyclodextrin-modified PPI dendrimers of the second generation (G2) modified with 31 % β-cyclodextrin (CD-PPI(G2) 8,500 g/mol) and of the fourth generation (G4) modified with 7 % β-cyclodextrin (CD-PPI(G4) 13,350 g/mol) are used to prepare the hybrid polyplexes of the invention.

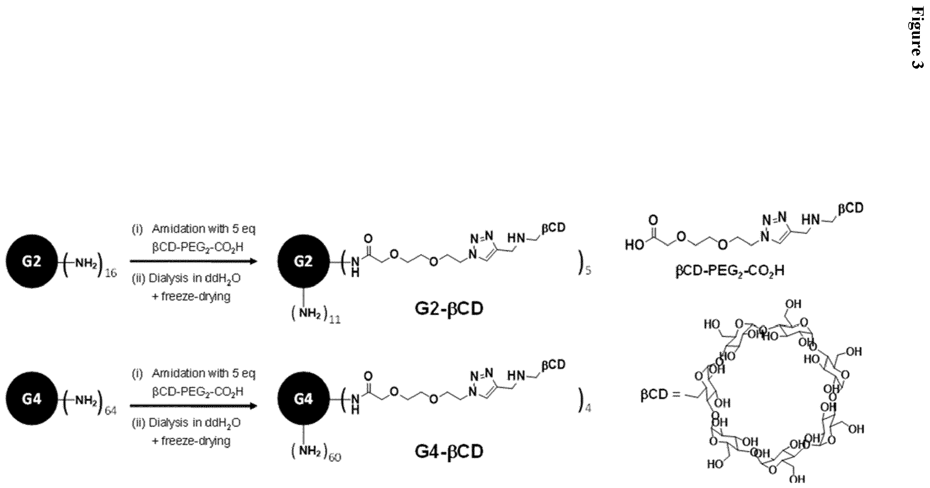

Most preferably, the β-cyclodextrin-modified PPI dendrimers used in the hybrid polyplexes of the invention are those as shown in Figure 3.

It has been further shown that the concentration of the PPIs and the DNA:PPI mass ratio may affect biocompatibility and cytotoxicity of the delivery system of the invention. To avoid cytotoxicity and non-specific transfection efficacy, concentrations of PPIs below 2.5 µM and DNA:cyclodextrin PPI mass ratio of 5:1 did not affect viability of tested cells and met the criterion of biocompatibility. Accordingly, in one embodiment of the invention, the delivery system of the invention comprises hybrid polyplexes as described herein above, wherein the DNA:cyclodextrin PPI mass ratios is 5:1 or lower, preferably 5:1 or 4:1, more preferably 3:1, 2:1 or 1:1 in the provided dendriplexes. In a further preferred embodiment, the delivery system of the invention comprises cyclodextrin-PPI and mal19-PPI dendrimers with a mass PPI/DNA ratio of 5:1:1. Thereby, an efficient endosomal release of the payload-DNA from its complexation in said hybrid polyplexes is ensured.

Preferred examples of the composition of the delivery system of the invention are as follows:
i) A delivery system for targeted delivery of nucleic acid-based therapeutics, comprising
   - an avidin core, wherein the avidin core consists of a avidin, neutravidin or streptavidin molecule;
   - at least one biotinylated targeting molecule selected from the group consisting of a natural or artificial protein-ligand, aptamer, antibody, or antibody single-chain variable fragment (scFv),
   - at least one therapeutically active nucleic acid,
   wherein said at least one therapeutically active nucleic acid is complexed with a biotinylated hybrid polyplex comprising maltose-PPI and cyclodextrin-PPI, wherein PPI is defined as poly(propylene imine) (PPI) and wherein said at least one targeting molecule and said biotinylated hybrid polyplex are conjugated to said avidin core.
ii) A delivery system for targeted delivery of nucleic acid-based therapeutics, comprising
   - an avidin core, wherein the avidin core consists of a avidin, neutravidin or streptavidin molecule;
   - at least one biotinylated antibody single-chain variable fragment (scFv) as the targeting molecule;
   - at least one therapeutically active nucleic acid, which is selected from the group consisting of CpG oligonucleotides, ssDNA, dsDNA, ssRNA or dsRNA;
   wherein said at least one therapeutically active nucleic acid is complexed with a biotinylated hybrid polyplex comprising maltose-PPI and cyclodextrin-PPI, wherein PPI is defined as poly(propylene imine) (PPI) and wherein said at least one targeting molecule and said biotinylated hybrid polyplex are conjugated to said avidin core.
iii) A delivery system for targeted delivery of nucleic acid-based therapeutics, comprising
   - an avidin core, wherein the avidin core consists of a avidin, neutravidin or streptavidin molecule;
   - at least one biotinylated antibody single-chain variable fragment (scFv) as the targeting molecule;
   - at least one therapeutically active nucleic acid, which is selected from plasmid DNA or minicircle-DNA;
   wherein said at least one therapeutically active nucleic acid is complexed with a biotinylated hybrid polyplex comprising maltose-PPI and cyclodextrin-PPI, wherein PPI is defined as poly(propylene imine) (PPI) and wherein said at least one targeting molecule and said biotinylated hybrid polyplex are conjugated to said avidin core..

In a preferred embodiment of the invention, said plasmid DNA or minicircle-DNA encodes the human tumor suppressor gene *p53* (SEQ ID NO: 11).

As a transcription factor, the human tumor suppressor p53 regulates the expression of genes involved in cell cycle control, the induction of apoptosis (programmed cell death) or DNA repair after DNA damage. Because of this property, p53 is referred to in the literature as the "guardian of the genome". The special medical importance can be explained by the finding that p53 is mutated in 50% of all human tumors. The loss of p53 function therefore plays a critical role in the development of cancer.

The inventors have successfully demonstrated in an experimental gene therapy approach, that targeted transposition of a codon-optimized p53 into p53 deficient HCT116p53-/-/PSCA cells demonstrated decreased clonogenic survival when compared to mock controls (see working examples). Noteworthy, p53 transposition in PTEN-deficient H4-PSCA glioma cells caused nearly complete loss of clonogenic survival. These results demonstrate the feasibility of the delivery system of the invention combining tumor-targeting hybrid polyplexes and Sleeping Beauty gene transposition, which due to the modular design can be extended to other target genes and tumor entities.
iv) A delivery system for targeted delivery of nucleic acid-based therapeutics, comprising
- an avidin core, wherein the avidin core consists of a avidin, neutravidin or streptavidin molecule;
- at least one biotinylated antibody single-chain variable fragment (scFv) - linker - BAP as the targeting molecule;
- at least one therapeutically active nucleic acid, which is selected from the group consisting of CpG oligonucleotides, ssDNA, dsDNA, ssRNA or dsRNA;
wherein said at least one therapeutically active nucleic acid is complexed with a biotinylated hybrid polyplex comprising maltose-PPI and cyclodextrin-PPI, wherein PPI is defined as poly(propylene imine) (PPI) and wherein said at least one targeting molecule and said biotinylated hybrid polyplex are conjugated to said avidin core.

In a preferred embodiment of the invention, said antibody single-chain variable fragment (scFv) in said scFv - linker - BAP targeting molecule is scFv(h-AM-1) (SEQ ID NO: 1).
v) A delivery system for targeted delivery of nucleic acid-based therapeutics, comprising
- an avidin core, wherein the avidin core consists of a avidin, neutravidin or streptavidin molecule;
- at least one biotinylated antibody single-chain variable fragment (scFv) - linker - BAP as the targeting molecule;
- at least one therapeutically active nucleic acid, which is selected from plasmid DNA and minicircle-DNA;
wherein said at least one therapeutically active nucleic acid is complexed with a biotinylated hybrid polyplex comprising maltose-PPI and cyclodextrin-PPI, wherein PPI is defined as poly(propylene imine) (PPI) and wherein said at least one targeting molecule and said biotinylated hybrid polyplex are conjugated to said avidin core.

In a preferred embodiment of the invention, said plasmid DNA or minicircle-DNA encodes the human tumor suppressor gene *p53* (SEQ ID NO: 11).

In a further preferred embodiment of the invention, said antibody single-chain variable fragment (scFv) in said scFv - linker - BAP targeting molecule is scFv(h-AM-1) (SEQ ID NO: 1).
vi) A delivery system for targeted delivery of nucleic acid-based therapeutics, comprising
- an avidin core, wherein the avidin core consists of a avidin, neutravidin or streptavidin molecule;
- at least one biotinylated antibody single-chain variable fragment(scFV) - linker peptide - BAP as the targeting molecule;
   wherein
      said BAP is selected from the group consisting of:
      ∘ 
MKLKVTVNGTAYDVDVDVDKSHENPMGTILFGGGTGGAPAPAAGGA
GAGKAGEGEIPAPLAGTVSKILVKEGDTVKAGQTVLVLEAMKMETEIN
APTDGKVEKVLVKERDAVQGGQGLIKIGDLEL (SEQ ID NO. 2);
      ∘ 
VLRSPMPGVVVAVSVKPGDAVAEGQEICVIEAMKMQNSMTAGKTGT
VKSVHCQAGDTVGEGDLLVELE (SEQ ID NO: 3);
      ∘ LX₁X₂IFEAQKIEWR (SEQ ID NO: 4), wherein
         X₁ = any amino acid; and
         X₂ = is any amino acid except L, V, I, W, F or Y;
      ∘ GLNDIFEAQKIEWHE (SEQ ID NO. 5);
      ∘ ALNDIFEAQKIEWHA (SEQ ID NO: 6);
      ∘ MAGGLNDIFEAQKIEWHEDTGGS (SEQ ID NO. 7);
      ∘ MSGLNDIFEAQKIEWHEGAPSSR (SEQ ID NO: 8); and
      ∘ LHHILDAQKMVWNHR (SEQ ID NO: 9); and
   wherein said linker peptide is selected from the group consisting of
      ∘ two amino acids, such as GS;
      ∘ 6 amino acids; and
      ∘ 10 amino acids, such as the c-myc tag having the amino acid sequence of EQKLISEEDL (SEQ ID NO: 10);
- at least one therapeutically active nucleic acid, which is selected from the group consisting of CpG oligonucleotides, ssDNA, dsDNA, ssRNA or dsRNA;
wherein said at least one therapeutically active nucleic acid is complexed with a biotinylated hybrid polyplex comprising maltose-PPI and cyclodextrin-PPI, wherein PPI is defined as poly(propylene imine) (PPI) and wherein said at least one targeting molecule and said biotinylated hybrid polyplex are conjugated to said avidin core.

In a preferred embodiment of the invention, said antibody single-chain variable fragment (scFv) in said scFv - linker - BAP targeting molecule is scFv(h-AM-1) (SEQ ID NO: 1).
vii) A delivery system for targeted delivery of nucleic acid-based therapeutics, comprising
   - an avidin core, wherein the avidin core consists of a avidin or neutravidin molecule;
   - at least one biotinylated antibody single-chain variable fragment (scFV) - linker peptide - BAP as the targeting molecule;
      wherein
         said at least one antibody single-chain variable fragment (scFV) is scFv(AM1) (SEQ ID NO: 1)
         said BAP is selected from
            ∘ 
MKLKVTVNGTAYDVDVDVDKSHENPMGTILFGGGTGGAPAPAAG
GAGAGKAGEGEIPAPLAGTVSKILVKEGDTVKAGQTVLVLEAMKM
ETEINAPTDGKVEKVLVKERDAVQGGQGLIKIGDLEL (SEQ ID NO.
 2); and
         wherein said linker peptide is c-myc tag having the amino acid sequence of EQKLISEEDL (SEQ ID NO: 10);
   - at least one therapeutically active nucleic acid, which is selected from the group
      consisting of CpG oligonucleotides, ssDNA, dsDNA, ssRNA or dsRNA;
   wherein said at least one therapeutically active nucleic acid is complexed with a biotinylated hybrid polyplex comprising maltose-PPI and cyclodextrin-PPI, wherein PPI is defined as poly(propylene imine) (PPI) and wherein said at least one targeting molecule and said biotinylated hybrid polyplex are conjugated to said avidin core.
viii) A delivery system for targeted delivery of nucleic acid based therapeutics, comprising
   - an avidin core, wherein the avidin core consists of a avidin or neutravidin molecule;
   - at least one biotinylated antibody single-chain variable fragment (scFV) - linker peptide - BAP as the targeting molecule;
      wherein
         said at least one antibody single-chain variable fragment (scFV) is scFv(AM1) (SEQ ID NO: 1)
         said BAP is selected from
            ∘ 
MKLKVTVNGTAYDVDVDVDKSHENPMGTILFGGGTGGAPAPAAG
GAGAGKAGEGEIPAPLAGTVSKILVKEGDTVKAGQTVLVLEAMKM
ETEINAPTDGKVEKVLVKERDAVQGGQGLIKIGDLEL (SEQ ID NO.
 2); and
      wherein said linker peptide is c-myc tag having the amino acid sequence of EQKLISEEDL (SEQ ID NO: 10);
   - at least one therapeutically active nucleic acid, which is selected from plasmid DNA and minicircle-DNAs, which are preferably encoding the human tumor suppressor gene *p53* (SEQ ID NO: 11);
   wherein said at least one therapeutically active nucleic acid is complexed with a biotinylated hybrid polyplex comprising maltose-PPI and cyclodextrin-PPI, wherein PPI is defined as poly(propylene imine) (PPI) and wherein said at least one targeting molecule and said biotinylated hybrid polyplex are conjugated to said avidin core.
ix) A delivery system for targeted delivery of nucleic acid based therapeutics, comprising
   - a neutravidin molecule as the avidine core;
   - at least one scFv(AM1) (SEQ ID NO: 1) antibody single-chain variable fragment (scFv) - linker peptide - BAP as the targeting molecule, which is biotinylated;
      wherein
         said BAP is 
MKLKVTVNGTAYDVDVDVDKSHENPMGTILFGGGTGGAPAPAAG
GAGAGKAGEGEIPAPLAGTVSKILVKEGDTVKAGQTVLVLEAMKM
ETEINAPTDGKVEKVLVKERDAVQGGQGLIKIGDLEL (SEQ ID NO.
 2); and
      wherein said linker peptide is c-myc tag having the amino acid sequence of EQKLISEEDL (SEQ ID NO: 10);
   - at least one therapeutically active nucleic acid, which is selected from at least one plasmid DNA molecule and at least one minicircle DNA molecule, wherein said at least one plasmid DNA molecule or said at least one minicircle DNA molecule is preferably encoding p53 (SEQ ID NO. 11);
      wherein said at least one therapeutically active nucleic acid is complexed with a biotinylated hybrid polyplex comprising maltose-PPI and cyclodextrin-PPI, wherein PPI is defined as poly(propylene imine) (PPI) and wherein said at least one targeting molecule and said biotinylated hybrid polyplex are conjugated to said avidin core.

In a further preferred embodiment of the invention, the delivery system according to any one of items i) to ix) comprises
a) one antibody single-chain variable fragment and three therapeutically active nucleic acids, or
b) two antibody single-chain variable fragments and two therapeutically active nucleic acids, or
c) three antibody single-chain variable fragments and one therapeutically active nucleic acid.

Further preferably, the delivery system according to any one of items i) to ix) may comprise a mixture of components a), b) and c) above, wherein component b) statistically forms the main share in said mixture.

In a further embodiment, the invention provides a process for the assembly of the delivery system according to the invention comprising the steps of:
a) preparing conjugates comprising biotinylated targeting molecules conjugated to avidin, neutravidin or streptavidin,
b) preparing cyclodextrin-PPI dendrimers,
c) preparing maltose-PPI-biotin dendrimers;
d) incubating maltose-PPI-biotin dendrimers with the conjugates resulting from step a), wherein the maltose-PPI has a cationic charge;
e) binding the maltose-PPI-biotin dendrimers to the conjugates resulting from step a);
f) separately, incubating cyclodextrin-PPI with nucleic acid, wherein cyclodextrin-PPI-nucleic acid dendrimers are formed, which have a weak anionic charge,
g) incubating the complexes resulting from step e) with the cyclodextrin-PPI-nucleic acid dendrimers of step f), and
h) formation, through ionic interaction, of a tumor-targeting delivery system, comprising biotinylated targeting molecules and hybrid polyplexes comprising maltose-PPI-biotin/cyclodextrin-PPI dendrimers which contain the nucleic acid payload, wherein said biotinylated targeting molecules and said hybrid polyplexes are bound to the avidin/neutravidin core.

The advantages and advantageous embodiments described for the delivery system above equally apply to the process for the assembly of the delivery system such that it shall be referred to the above.

In a preferred embodiment, the invention therefore provides a process for the assembly of the delivery system according to the invention comprising the steps of:
a) preparing conjugates comprising biotinylated scFv-linker peptide-BAP conjugated to avidin, neutravidin or streptavidin;
b) preparing cyclodextrin-PPI dendrimers,
c) preparing maltose-PPI-biotin dendrimers;
d) incubating maltose-PPI-biotin dendrimers with the conjugates resulting from step a), wherein the maltose-PPI has a cationic charge;
e) binding the maltose-PPI-biotin dendrimers to the conjugates resulting from step a);
f) separately, incubating cyclodextrin-PPI dendrimers with a nucleic acid, wherein cyclodextrin-PPI-nucleic acid dendrimers are formed, which have a weak anionic charge,
g) incubating the complexes resulting from step e) with the cyclodextrin-PPI-nucleic acid dendrimers of step f), and
h) formation, through ionic interaction, of a tumor-targeting delivery system, comprising biotinylated scFv-linker peptide-BAP and hybrid polyplexes comprising maltose-PPI-biotin/cyclodextrin-PPI dendrimers which contain the nucleic acid payload, wherein said biotinylated scFv-linker peptide-BAP and said hybrid polyplexes are bound to the avidin/neutravidin core.

The order of steps a) to g) is generally interchangeable. However, it is preferred according to the invention that steps a) to g) are performed in the order described above.

The site-specific mono-biotinlyation of biological molecules, such as the antibody single-chain variable fragments and therapeutically active nucleic acids of the invention can be done by any conventional method.

Biotinylation is the process of attaching biotin to proteins and other macromolecules. Biotinylation reagents are available for targeting specific functional groups or residues, including primary amines, sulfhydryls, carboxyls and carbohydrates. Photoreactive biotin compounds that react nonspecifically upon exposure to ultraviolet (UV) light are also available and expand the scope of the molecules that may be biotinylated. The variety of biotinylation reagents with different functional group specificities is extremely useful, allowing one to choose a reagent that does not inactivate the target macromolecule. Besides functional group specificity, biotinylation reagents are available with different solubility characteristics to focus biotinylation to distinct microenvironments either inside or outside cells. Cleavable or reversible biotinylation reagents enable the specific elution of biotinylated molecules from biotin-binding proteins. The variability of these reagents substantially expands the range of applications for avidin-biotin chemistry. The bond formation between biotin and avidin is very rapid, and once formed, it is unaffected by extremes in pH, temperature, organic solvents and other denaturing agents. Biotinylation is most commonly performed through chemical means, but enzymatic methods are also available.

For biotinylation of the antibody single-chain variable fragments according to the invention, enzymatic approaches that can be performed both *in vitro* and *in vivo* are preferred. In particular, enzymatic methods are preferred, in which a bacterial biotin ligase and an exogenously expressed protein of interest are co-expressed and in which the expressed protein is modified to carry a biotin acceptor peptide, which provides a more uniform biotinylation (site-specific biotinylation) than chemical methods. Most preferably, the present invention uses an enzymatic natural machinery, i.e. the *E. coli* enzyme BirA, to achieve precise biotin modification. The natural substrate of BirA is the Biotin Carboxyl Carrier Protein (BCCP), requiring fusion of at least 75 residues to the target protein. However, phage display selection enabled the development of the AviTag (also known as the Biotin Acceptor Peptide, BAP), which is superior to BCCP as a BirA substrate but only 15 amino acids in length, so extending the range of protein sites amenable to site-specific enzymatic biotinylation. Other BAPs, which are substrates of the *E*. *coli* enzyme BirA ligase, are selected from the group consisting of:
- 
MKLKVTVNGTAYDVDVDVDKSHENPMGTILFGGGTGGAPAPAAGGA
GAGKAGEGEIPAPLAGTVSKILVKEGDTVKAGQTVLVLEAMKMETEIN
APTDGKVEKVLVKERDAVQGGQGLIKIGDLEL SEQ ID NO. 2);
-

(VLRSPMPGVVVAVSVKPGDAVAEGQEICVIEAMKMQNSMTAGKTGT
VKSVHCQAGDTVGEGDLLVELE, SEQ ID NO: 3);

∘ LX₁X₂IFEAQKIEWR (SEQ ID NO: 4), wherein
   X₁ = any amino acid; and
   X₂ = is any amino acid except L, V, I, W, F or Y;
∘ GLNDIFEAQKIEWHE (SEQ ID NO. 5);
∘ ALNDIFEAQKIEWHA (SEQ ID NO: 6);
∘ MAGGLNDIFEAQKIEWHEDTGGS (SEQ ID NO. 7);
   MSGLNDIFEAQKIEWHEGAPSSR (SEQ ID NO: 8); and
∘ LHHILDAQKMVWNHR (SEQ ID NO: 9).

Enzymatic biotinylation with *E. coli* biotin ligase (BirA) is highly specific in covalently attaching biotin to the BAP, giving a homogeneous product with high yield. The BAP can conveniently be added genetically at the N-terminus, C-terminus or in exposed loops of a target protein. BirA can biotinylate substrate peptides specifically in the cytosol, secretory pathway, and at the cell surface in mammalian and invertebrate systems. Biotinylation of purified proteins has been applied in a wide range of areas of biochemistry and cell biology. An important advance in BirA labeling is its use for electron microscopy. Biotin ligase from *E. coli* or other species can also ligate to a peptide tag biotin analogs, including desthiobiotin for reversible streptavidin binding, or analogs containing functional groups for bio-orthogonal reaction: keto, azido and alkyne groups. Engineering of streptavidin is important in extending the usefulness of BirA-labeling; in particular variants with controlled valency (e.g. monovalent streptavidin, mSA), enabling precise control over assembly of biotin conjugates.

The tumor targeting polyplexes of step h) represent the embodiment of the delivery system according to the invention, in which the therapeutically active nucleic acid is represented by a dsDNA. The formation of the delivery system of step h) suitably occurs through ionic interaction.

Biotinylated scFv-linker peptide-BAP and ma119-PPI-biotin form stable complexes with avidin in a 1:1 to 4:1 stoichiometry, preferably in a 2:1 stoichiometry.

As above discussed for the delivery system of the invention, the cyclodextrin-PPI dendrimers are preferably 2nd generation PPI dendrimers and 4th generation PPI dendrimers, bearing 31 % and 7 % coupled cyclodextrin to peripheral amine groups. Further preferably, for generation of dendriplexes mass ratio of 2nd and 4th generation cyclodextrin PPIs to DNA was 5:1. For final assembly into the delivery system of the invention, hybrid polyplexes were incubated with preformed targeting conjugates comprising scFv-linker peptide-BAP : avidin or neutravidin or streptavidin : mal19-PPI-biotin at a molar ratio of 2:1:1.

The invention further relates to a delivery system for targeted delivery of nucleic acid-based therapeutics, which is obtainable by the processes according to the invention.

The delivery system for targeted delivery of nucleic acid based therapeutics can further be comprised in a pharmaceutical composition together with at least one pharmaceutically acceptable carrier or diluent.

The pharmaceutical compositions may be formulated with pharmaceutically acceptable carriers or diluents as well as any other known adjuvants and excipients in accordance with conventional techniques such as those disclosed in Remington: The Science and Practice of Pharmacy, 21th Edition, Gennaro, Ed., Mack Publishing Co., Easton, PA, 2005.

The pharmaceutically acceptable carriers or diluents as well as any other known adjuvants and excipients should be suitable for the chosen delivery system of the present invention and the chosen mode of administration. Suitability for carriers and other components of pharmaceutical compositions is determined based on the lack of significant negative impact on the desired biological properties of the chosen delivery system or pharmaceutical composition of the present invention (e.g., less than a substantial impact (10 % or less relative inhibition, 5 % or less relative inhibition, etc.)) on antigen binding.

A pharmaceutical composition of the present invention may also include diluents, fillers, salts, buffers, detergents (e.g., a nonionic detergent, such as Tween-20 or Tween-80), stabilizers (e.g., sugars or protein-free amino acids), preservatives, tissue fixatives, solubilizers, and/or other materials suitable for inclusion in a pharmaceutical composition.

The actual dosage levels of the active ingredients in the pharmaceutical compositions of the present invention may be varied so as to obtain an amount of the active ingredient which is effective to achieve the desired therapeutic response for a particular patient, composition, and mode of administration. The selected dosage level will depend upon a variety of pharmacokinetic factors including the activity of the particular compositions of the present invention employed, or the amide thereof, the route of administration, the time of administration, the rate of excretion of the particular delivery system being employed, the duration of the treatment, other drugs, compounds and/or materials used in combination with the particular compositions employed, the age, sex, weight, condition, general health and prior medical history of the patient being treated, and like factors well known in the medical arts.

The pharmaceutical composition may be administered by any suitable route and mode. Suitable routes of administering a delivery system of the present invention in vivo and in vitro are well known in the art and may be selected by those of ordinary skill in the art.

In one embodiment, a pharmaceutical composition of the present invention is administered parenterally.

The phrases "parenteral administration" and "administered parenterally" as used herein means modes of administration other than enteral and topical administration, usually by injection, and include epidermal, intravenous, intramuscular, intraarterial, intrathecal, intracapsular, intraorbital, intracardiac, intradermal, intraperitoneal, intratendinous, transtracheal, subcutaneous, subcuticular, intraarticular, subcapsular, subarachnoid, intraspinal, intracranial, intrathoracic, epidural and intrasternal injection and infusion.

In one embodiment the pharmaceutical composition of the invention is administered by intravenous or subcutaneous injection or infusion.

Pharmaceutically acceptable carriers include any and all suitable solvents, dispersion media, coatings, antibacterial and antifungal agents, isotonicity agents, antioxidants and absorption delaying agents, and the like that are physiologically compatible with a delivery system of the present invention.

Examples of suitable aqueous and nonaqueous carriers which may be employed in the pharmaceutical compositions of the present invention include water, saline, phosphate buffered saline, ethanol, dextrose, polyols (such as glycerol, propylene glycol, polyethylene glycol, and the like), and suitable mixtures thereof, vegetable oils, such as olive oil, corn oil, peanut oil, cottonseed oil, and sesame oil, carboxymethyl cellulose colloidal solutions, tragacanth gum and injectable organic esters, such as ethyl oleate, and/or various buffers. Other carriers are well known in the pharmaceutical arts.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active delivery system, use thereof in the pharmaceutical compositions of the present invention is contemplated.

Proper fluidity may be maintained, for example, by the use of coating materials, such as lecithin, by the maintenance of the required particle size in the case of dispersions, and by the use of surfactants.

Pharmaceutical compositions of the present invention may also comprise pharmaceutically acceptable antioxidants for instance (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha- tocopherol, and the like; and (3) metal chelating agents, such as citric acid, ethylenediamine tetraacetic acid (EDTA), sorbitol, tartaric acid, phosphoric acid, and the like.

Pharmaceutical compositions of the present invention may also comprise isotonicity agents, such as sugars, polyalcohols, such as mannitol, sorbitol, glycerol or sodium chloride in the compositions.

The pharmaceutical compositions of the present invention may also contain one or more adjuvants appropriate for the chosen route of administration such as preservatives, wetting agents, emulsifying agents, dispersing agents, preservatives or buffers, which may enhance the shelf life or effectiveness of the pharmaceutical composition. The delivery systems of the present invention may be prepared with carriers that will protect the delivery system against rapid release, such as a controlled release formulation, including implants, transdermal patches, and microencapsulated delivery systems. Such carriers may include gelatin, glyceryl monostearate, glyceryl distearate, biodegradable, biocompatible polymers such as ethylene vinyl acetate, polyanhydrides, polyglycolic acid, collagen, polyorthoesters, and polylactic acid alone or with a wax, or other materials well known in the art. Methods for the preparation of such formulations are generally known to those skilled in the art. See, e.g., Sustained and Controlled Release Drug Delivery Systems, J. R. Robinson, ed., Marcel Dekker, Inc., New York, 1978.

In one embodiment, the antibodies of the present invention may be formulated to ensure proper distribution in vivo. Pharmaceutically acceptable carriers for parenteral administration include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the delivery system, use thereof in the pharmaceutical compositions of the present invention is contemplated.

Pharmaceutical compositions for injection must typically be sterile and stable under the conditions of manufacture and storage. The composition may be formulated as a solution, microemulsion, liposome, or other ordered structure suitable to high drug concentration. The carrier may be a aqueous or non-aqueous solvent or dispersion medium containing for instance water, ethanol, polyols (such as glycerol, propylene glycol, polyethylene glycol, and the like), and suitable mixtures thereof, vegetable oils, such as olive oil, and injectable organic esters, such as ethyl oleate. The proper fluidity may be maintained, for example, by the use of a coating such as lecithin, by the maintenance of the required particle size in the case of dispersion and by the use of surfactants. In many cases, it will be preferable to include isotonic agents, for example, sugars, polyalcohols such as glycerol, mannitol, sorbitol, or sodium chloride in the composition. Prolonged absorption of the injectable compositions may be brought about by including in the composition an agent that delays absorption, for example, monostearate salts and gelatin. Sterile injectable solutions may be prepared by incorporating the delivery system in the required amount in an appropriate solvent with one or a combination of ingredients e.g. as enumerated above, as required, followed by sterilization microfiltration. Generally, dispersions are prepared by incorporating the delivery system into a sterile vehicle that contains a basic dispersion medium and the required other ingredients e.g. from those enumerated above. In the case of sterile powders for the preparation of sterile injectable solutions, examples of methods of preparation are vacuum drying and freeze-drying (lyophilization) that yield a powder of the active ingredient plus any additional desired ingredient from a previously sterile-filtered solution thereof.

Sterile injectable solutions may be prepared by incorporating the delivery system in the required amount in an appropriate solvent with one or a combination of ingredients enumerated above, as required, followed by sterilization microfiltration. Generally, dispersions are prepared by incorporating the delivery system into a sterile vehicle that contains a basic dispersion medium and the required other ingredients from those enumerated above. In the case of sterile powders for the preparation of sterile injectable solutions, examples of methods of preparation are vacuum drying and freeze-drying (lyophilization) that yield a powder of the active ingredient plus any additional desired ingredient from a previously sterile-filtered solution thereof. Dosage regimens in the above methods of treatment and uses are adjusted to provide the optimum desired response (e.g., a therapeutic response). For example, a single bolus may be administered, several divided doses may be administered over time or the dose may be proportionally reduced or increased as indicated by the exigencies of the therapeutic situation. Parenteral compositions may be formulated in dosage unit form for ease of administration and uniformity of dosage. Dosage unit form as used herein refers to physically discrete units suited as unitary dosages for the subjects to be treated; each unit contains a predetermined quantity of delivery system calculated to produce the desired therapeutic effect in association with the required pharmaceutical carrier. The specification for the dosage unit forms of the present invention are dictated by and directly dependent on (a) the unique characteristics of the delivery system and the particular therapeutic effect to be achieved, and (b) the limitations inherent in the art of compounding such an delivery system for the treatment of sensitivity in individuals. The effective dosages and the dosage regimens for the delivery systems of the invention depend on the disease or condition to be treated and may be determined by the persons skilled in the art. An exemplary, non-limiting range for a therapeutically effective amount of an antibody of the present invention is about 0.1 -10 mg/kg/body weight, such as about 0.1-5 mg/kg/body weight, for example about 0.1-2 mg/kg/body weight, such as about 0.1-1 mg/kg/body weight, for instance about 0.15, about 0.2, about 0.5, about 1, about 1.5 or about 2 mg/kg/body weight.

A physician or veterinarian having ordinary skill in the art may readily determine and prescribe the effective amount of the pharmaceutical composition required. For example, the physician or veterinarian could start doses of the targeting bio-conjugates employed in the pharmaceutical composition at levels lower than that required in order to achieve the desired therapeutic effect and gradually increase the dosage until the desired effect is achieved. In general, a suitable daily dose of a composition of the present invention will be that amount of the delivery system which is the lowest dose effective to produce a therapeutic effect. Such an effective dose will generally depend upon the factors described above. Administration may e.g. be intravenous, intramuscular, intraperitoneal, or subcutaneous, and for instance administered proximal to the site of the target. If desired, the effective daily dose of a pharmaceutical composition may be administered as two, three, four, five, six or more sub-doses administered separately at appropriate intervals throughout the day, optionally, in unit dosage forms. While it is possible for a delivery system of the present invention to be administered alone, it is preferable to administer the delivery system as a pharmaceutical composition as described above. siRNA may be delivered for research purposes or to produce a change in a cell that is therapeutic. In vivo delivery of siRNA is useful for research reagents and for a variety of therapeutic, diagnostic, target validation, genomic discovery, genetic engineering, and pharmacogenomic applications. Herein, siRNA delivery resulting in inhibition of endogenous gene expression in tumor cells is disclosed. Levels of a reporter (marker) gene expression measured following delivery of a polynucleotide indicate a reasonable expectation of similar levels of gene expression following delivery of other polynucleotides. Levels of treatment considered beneficial by a person having ordinary skill in the art differ from disease to disease. The amount (dose) of delivery polymer and siRNA-conjugate that is to be administered can be determined empirically. Here, an effective knockdown of gene expression can be accomplished using 0.8-10 mg/kg weight implemented in the formulation of the biotin-immunoconjugates. In the same manner and with the same amounts, the delivery system of the present invention comprising hybrid polyplexes is especially feasible for the delivery of plasmid DNA and minicircle DNA.

As used herein, *in vivo* means that which takes place inside an organism and more specifically to a process performed in or on the living tissue of a whole, living multicellular organism (animal), such as a mammal, as opposed to a partial or dead one.

The delivery system for targeted delivery of nucleic acid based therapeutics and the pharmaceutical composition according to the invention are particularly useful in the treatment of proliferative diseases. Accordingly, the invention provides the delivery system for targeted delivery of nucleic acid based therapeutics and/or the pharmaceutical composition as described herein for use in the treatment of proliferative diseases.

In a further embodiment, the invention relates to method of treatment of proliferative diseases comprising the administration of a therapeutically effective dose of the delivery system for targeted delivery of nucleic acid based therapeutics and/or the pharmaceutical composition as described herein to a subject in need thereof.

In yet a further embodiment, the invention relates to the use of the delivery system for targeted delivery of nucleic acid based therapeutics and/or the pharmaceutical composition for the preparation of a medicament for the treatment of proliferative diseases.

Said proliferative diseases are for example primary tumors like glioblastoma multiforme, (GBM), pancreatic cancer, prostate cancer, urogenital tumors or metastatic cancers.

In a more preferred embodiment, said proliferative diseases, is selected from small cell lung cancer, small cell renal cancer, breast cancer, prostate cancer, bladder cancer and malignant glioma.

In a further preferred embodiment, the delivery system for targeted delivery of nucleic acid based therapeutics and/or the pharmaceutical composition are used in a combination therapy with other anti-tumor drugs. Preferred other anti-tumor drugs are EGF receptor inhibitors, such as tyrosine kinase inhibitors or monoclonal antibodies that slow down or halt cell growth. Suitable tyrosine kinase inhibitors for use in the combination therapy according to the invention are for example selected from gefitinib, erlotinib, afatinib and osimertinib for the treatment of lung cancer, and cetuximab for the treatment of colon cancer.

A suitable monoclonal antibody for use in the combination therapy according to the invention is for example CimaVax-EGF, an active vaccine targeting EGF as the major ligand of EGFR, which raises antibodies against EGF itself, thereby denying EGFR-dependent cancers of a proliferative stimulus.

Further suitable other anti-tumor drugs are nucleic agonists for endosomal and cytosolic pattern recognition receptors such as TLR3, TLR7, TLR9, RIG-I, and MDA5.

In a preferred embodiment and in order to overcome the obstacles of the prior art, a scFv-guided poly(propylene imine) (PPI)-based hybrid polyplex containing β-cyclodextrin was developed to efficiently deliver DNA to the cytoplasm of cells expressing the cognate receptor. More specifically, it was shown that a hybrid polyplex formation was achieved by a sequential conjugation of scFv-linker peptide-BAP biomolecules to neutravidin and to mono-biotinylated mal19-biotin and their subsequent electrostatic assembly to DNA complexed with β-cyclodextrin at defined stoichiometry. Compared to hybrid polyplexes conjugated to an unspecific control scFv-linker peptide-BAP, the generated specific polyplexes were able to bind to target cells expressing the cognate receptor and to exclusively deliver minicircle transposons encoding the gene of interest and minicircle DNA encoding Sleeping Beauty transposase by selective receptor-mediated endocytosis.

As a model targetable receptor for a proof of concept of this strategy, it was focused on prostate stem cell antigen (PSCA)-positive tumor cells. To reduce potential activation of Toll-like receptor 9 due to non-methylated bacterial plasmid sequences and to improve nanoparticle assembly and integrity, minicircle DNA instead of full plasmids were employed. To enable stable expression of transgenes, minicircle transposons encoding the gene of interest were transfected simultaneously with minicircles encoding Sleeping Beauty transposase.

Since inefficient endosomal escape of delivered DNA constitutes another critical issue for delivery of gene-encoding DNA and subsequent expression of the gene of interest in target cells, β-cyclodextrin-modified PPI were incorporated into polyplexes to enhance endosomal escape of the delivered DNA payload. This is a first example of efficient targeted delivery of gene-encoding DNA, in particular using a genetic transposition system, to cells expressing a cognate receptor using the biotin-avidin conjugation system, which, due to its modular composition, can also be further exploited towards other ligands or scFvs.

### Description of the drawings

The following figures are provided to illustrate various aspects of the invention. To that end, some of the figures contain schematic drawings and are not necessarily drawn to scale.

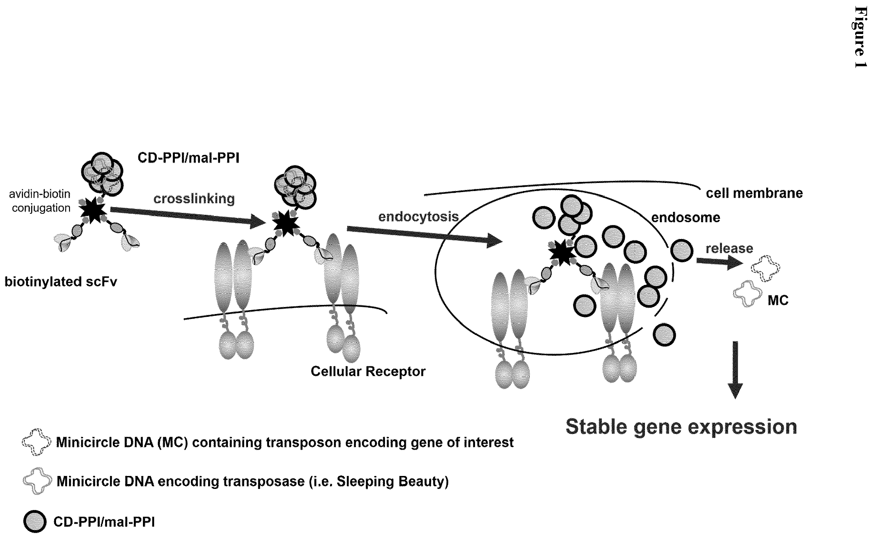

**Figure 1** shows a schematic drawing of the invention which is a biotin immunoconjugate for targeted transfection DNA, such as minicircle DNAs encoding a minicircle transposon encoding a gene of interest and Sleeping Beauty transposon, to cells by means of receptor-mediated endocytosis. The cross-linking of surface molecules leads to the internalization (endocytosis) of the maltose-PPI/cyclodextrin-PPI hybrid polyplexes into membrane containing vesicles (endosomes). After fusion with other endosomes, endosomal proton pumps decreases pH and together with cyclodextrin-proportion of PPIs facilitates release of minicircle DNAs into the cytosol of cells and subsequent transgene expression.

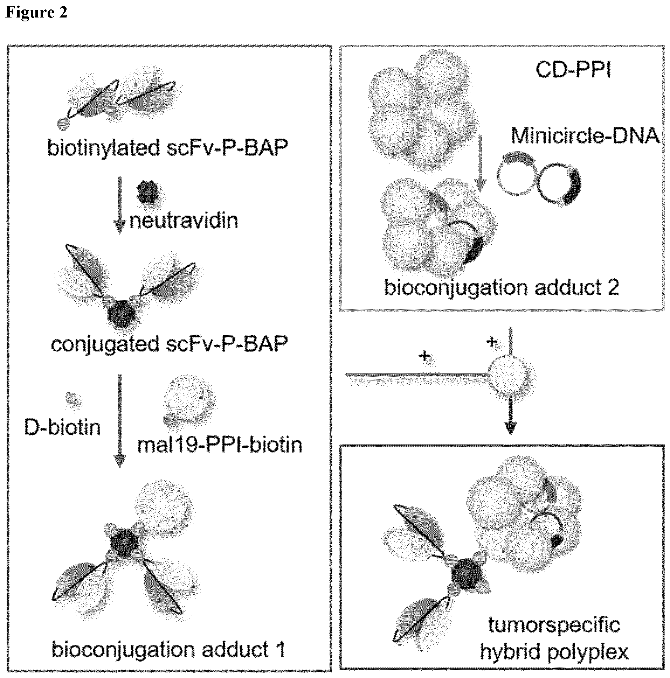

**Figure 2** shows components and assembly into hybrid maltose-PPI/cyclodextrin-PPI hybrid polyplexes.
**Figure 3** shows a schematic drawing of chemical synthesis of cyclodextrin-modified PPIs. Additionally, PEG-spacer and structure of cyclodextrin is depicted.

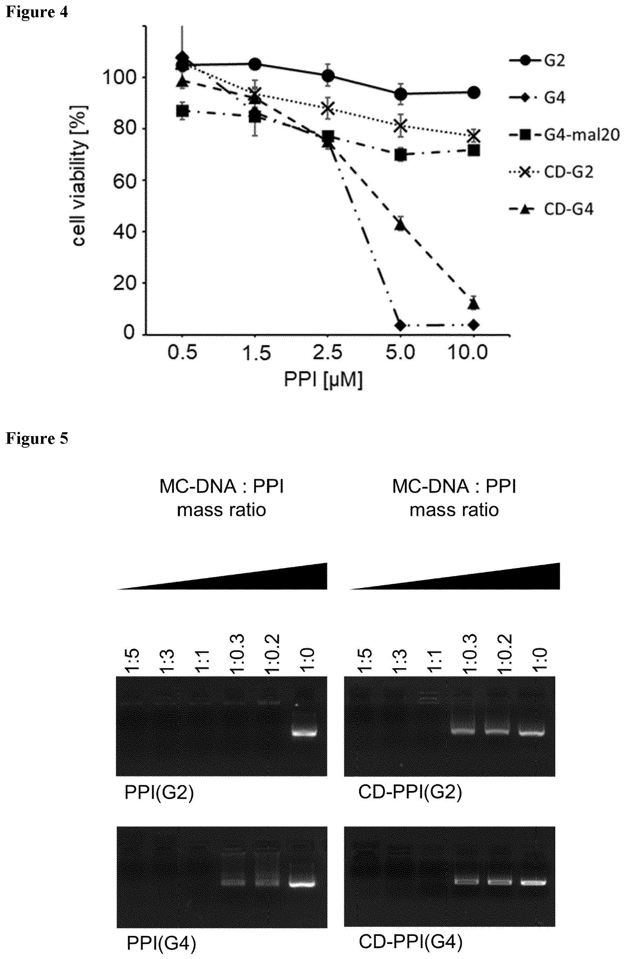

**Figure 4** shows the cytotoxic effects of increasing concentrations of different non-modified PPIs and cyclodextrin-modified PPIs of PPI generations G2 and G4 on 293T cells. Cell viability of 293T cells after treatment with mal19-PPI is also included in the analysis. All PPI molecules analyzed remained non-toxic at concentrations of 2.5 µM and below.
**Figure 5** shows the complexation of maltose-modified PPIs of generation G2 and G4 and of cyclodextrin-modified PPIs of generation G2 and G4 resulting in dendriplexes. Figures depict an agarose gel retention assay showing complexation of minicircle DNA with a size of approximately 3.7 kb with the different PPI-molecules at increasing PPI:DNA mass ratios. Non-complexed minicircle DNA served as control (1:0).

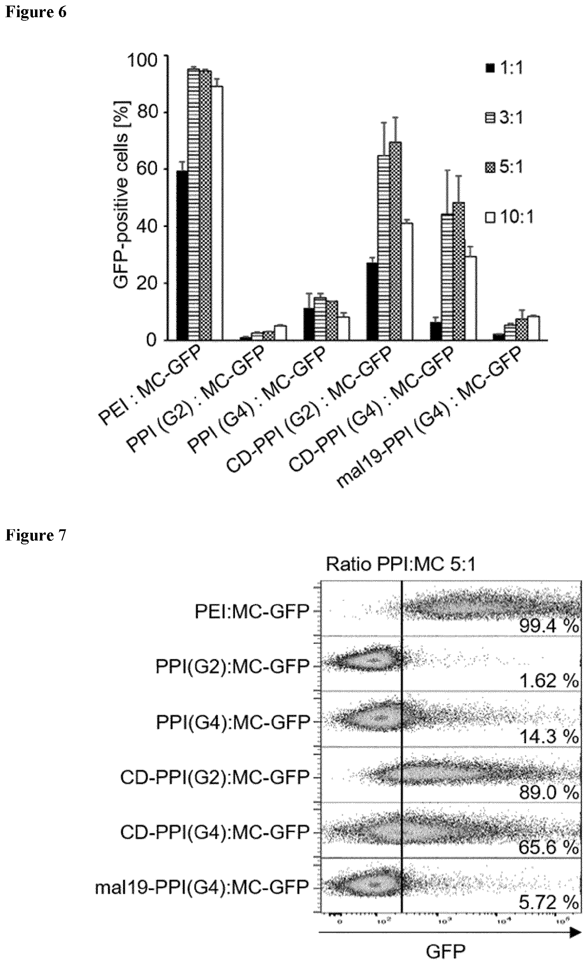

**Figure 6** shows transfection efficiencies of non-modified and cyclodextrin-modified PPIs at indicated PPI:DNA mass ratios. Plated 293T cells (100.000 cells/well) were transfected with 1 µg minicircle DNA encoding the reporter gene GFP. As positive control, a commercial PEI was included as transfection reagent. As a negative control, transfection-disabled mal19-PPI was complexed with 1 µg minicircle DNA and used for transfection of GFP into 293T cells.
**Figure 7** shows a representative transfection experiment as described in Fig. 6.

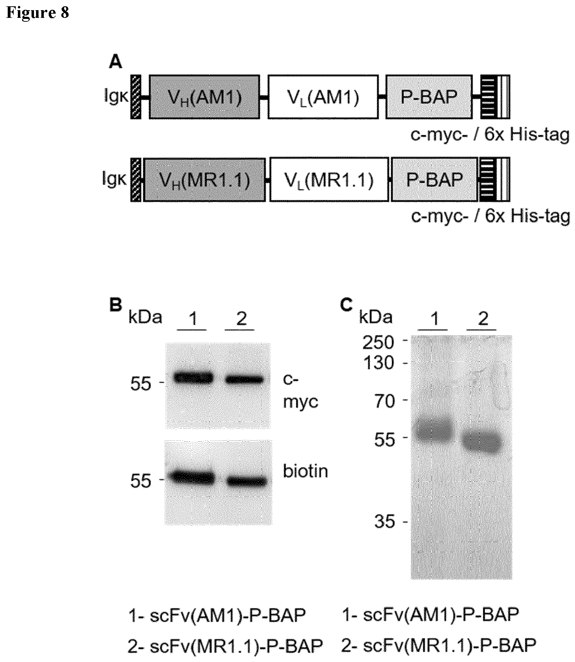

**Figure 8A** shows a schematic drawing of scFv-construct scFv(AM1)-P-BAP and of control scFv-(MR1.1)-P-BAP. The P-BAP domain enables intracellular biotinylation of the single-chain antibodies in 293T producer cells genetically engineered wit E. Coli biotin ligase. Fig. **8B** shows western blot analysis of affinity chromatography-purified scFv-BAPs. Equal amounts of scFv(AM1)-P-BAP and scFv-(MR1.1)-P-BAP were subjected to SDS-PAGE-electrophoresis, blotted onto nitrocellulose filters and probed with specific antibodies for c-myc-epitope and biotin followed by anti-horseradish peroxidase and enhanced chemiluminescence reaction. **Fig. 7C** shows Commassie Brilliant Blue-stained 12 % SDS-PAGE mini-gel showing purified scFv-BAP-proteins.

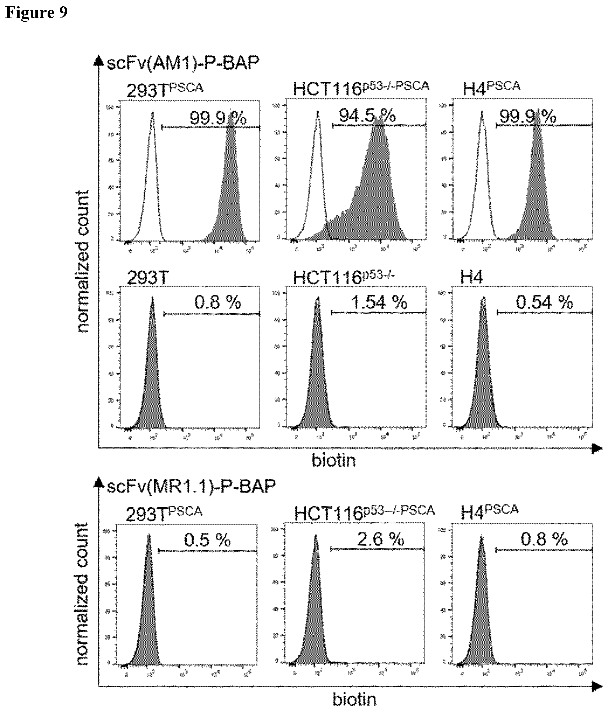

**Figure 9** demonstrates specific binding of the anti-PSCA scFv(AM1)-P-BAP on 293T-PSCA, HCT116^{p53-/-}-PSCA, and H4-PSCA target cells. No binding to cells is observed when using wild type cells as target cells or when employing the control scFv(MR1.1)-P-BAP targeting the neo-epitope EGFRvIII.

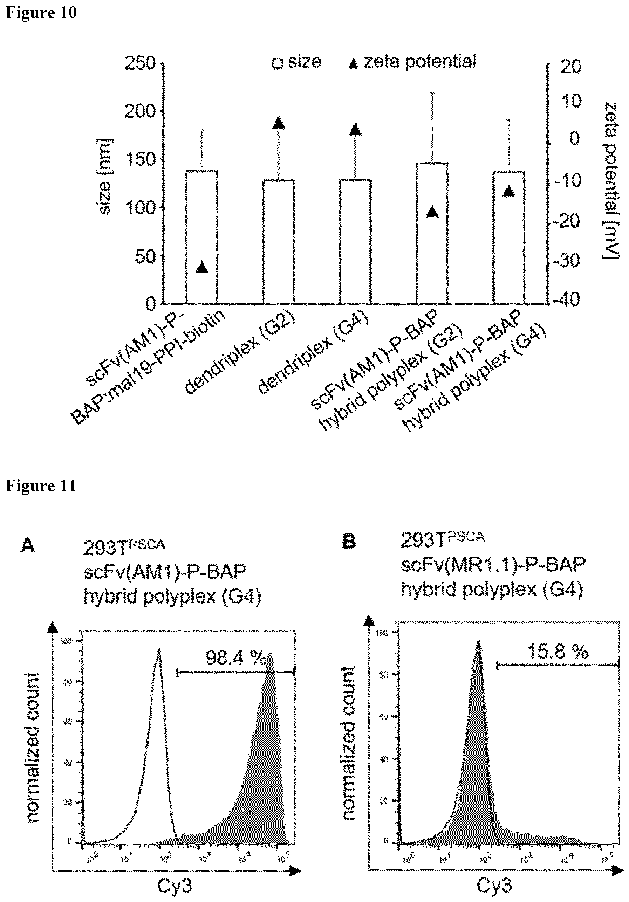

**Figure 10** shows assessment of particle sizes and zeta potential of scFv(AM1)-P-BAP-guided hybrid polyplexes.

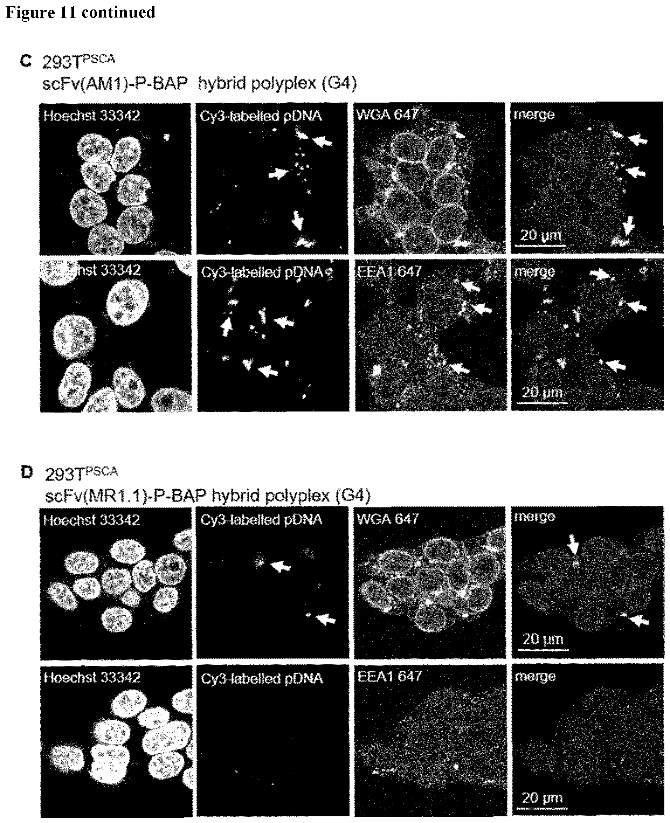

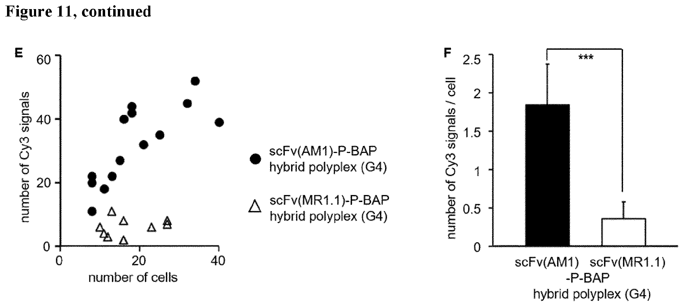

**Figure 11** shows targeted delivery of scFv(AM1)-P-BAP-guided hybrid polyplexes (G4) in PSCA-positive cells. 293T-PSCA cells were treated with scFv(AM1)-P-BAP **(A)** or scFv(MR1.1)-P-BAP hybrid polyplexes **(B)** containing Cy3-labelled plasmid DNA for 4 h (grey histograms). As control, untreated 293T-PSCA cells were included (open histograms). After washing of surface-bound antibodies employing heparin buffer, the remaining Cy3-signals representing internalized labelled plasmid DNA was measured by flow cytometry. C / D: Confocal laser scanning microscopy analysis of 293T-PSCA cells treated with scFv(AM1)-P-BAP or **(C)** or scFv(MR1.1)-P-BAP hybrid polyplexes **(D)** containing Cy3-labelled plasmid DNA. To visualize route of internalization, cells were additionally stained with wheat germ agglutinin or early endosomal marker EEA1. Arrows depict Cy3-labelled plasmid DNA or early endosomes. Analysis **(E)** and quantification **(F)** of Cy3-labelled plasmid DNA dots per image section or per cell. At least 14 image sections were analyzed (mean ± SD).

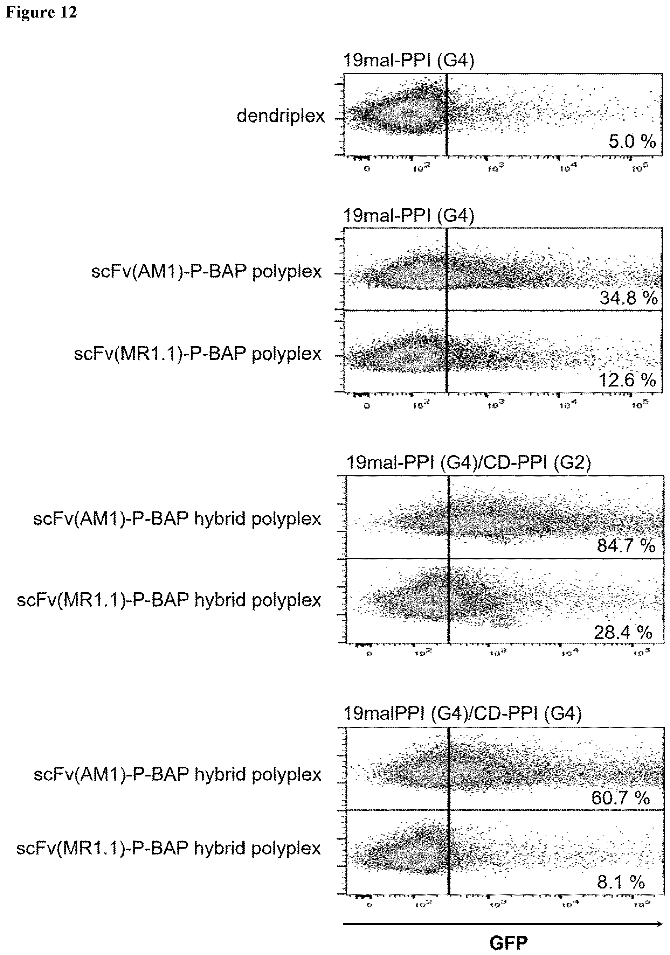

**Figure 12** shows representative results of DNA-transfection experiments in 293T-PSCA cells using indicated polyplexes containing different modified PPI molecules, PSCA-specific scFv(AM1)-P-BAP or control scFv(MR1.1)-P-BAP single chain antibodies. All depicted types of polyplexes contain a minicircle DNA encoding GFP. Transfection efficiencies were assessed by FACS-analyses of GFP.

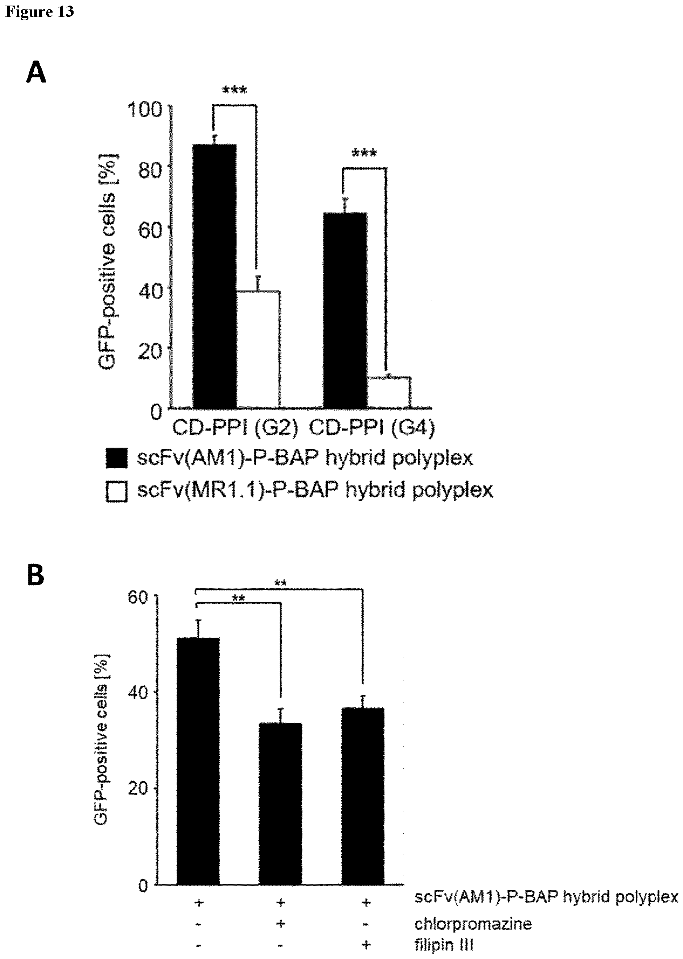

**Figure 13** **A** shows quantification of targeted transfection of minicircles encoding GFP into 293T-PSCA cells using hybrid mal-PPI/cyclodextrin-PPI containing polyplexes conjugated to scFv(AM1)-P-BAP, which is significantly higher when compared to hybrid polyplexes conjugated to the scFv(MR1.1)-P-BAP control. (mean ±SD, N=6, p<0.01). Figure 13 **B** depicts quantitative analysis of effects of endocytosis inhibitors chlorpromazine and filipin III (mean ±SD, N=6, p<0.05).

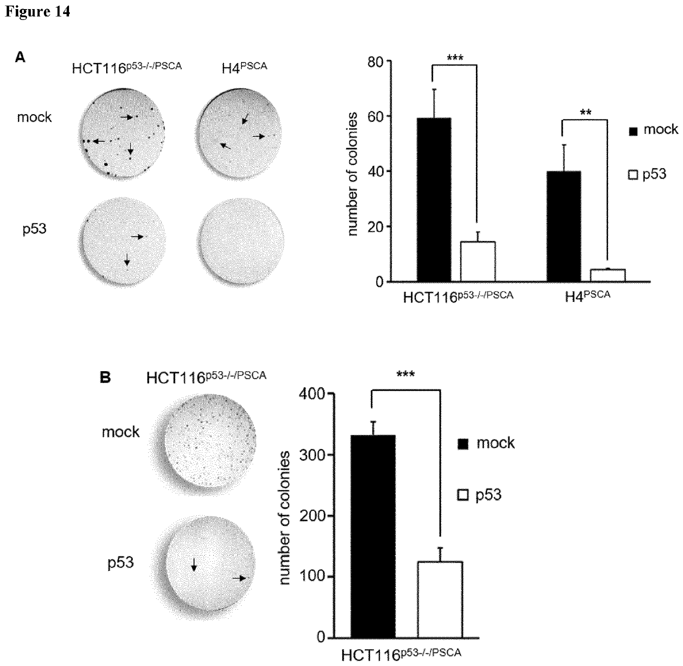

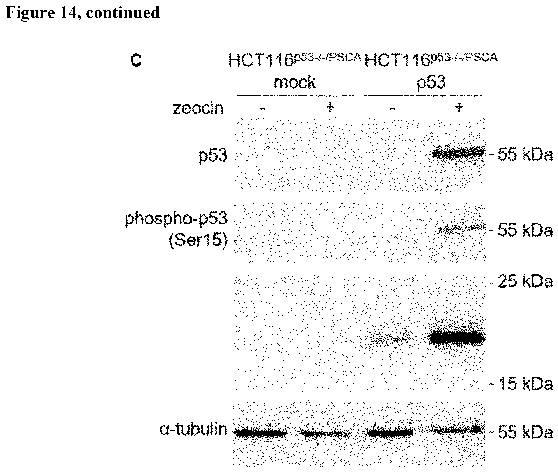

**Figure 14A** shows targeted simultaneous delivery of minicircle DNAs encoding a transposon encoding codon-optimized p53 and Sleeping Beauty transposase using hybrid polyplexes specific for PSCA. 2×10⁴ PSCA-positive HCT116^{p53-/-}PSCA cells and H4-PSCA glioma cells were plated in 30mm wells and after 18h were incubated with PSCA-specific polyplexes either enabling transposition of a transposon encoding p53 and puromycin resistance (p53) or a transposon only encoding for puromycin resistance (mock-transposon). 24h after treatment with hybrid polyplexes, cells were selected using 2 µg/ml puromycin and surviving clones were fixed at day 14, stained with crystal violet and quantified using ImageJ software. As depicted p53 transposition decreased clonogenic survival of HCT116^{p53-/-}PSCA cells and H4-PSCA glioma cells. In parallel experiments, pooled clones from chromosomally stable HCT116^{p53-/-}PSCA cells treated with p53 transposon could be passaged further. Yet, pooled clones from chromosomally instable H4-PSCA treated with p53 transposon succumbed to cells death (data not shown). As depicted in Fig. **14B** surviving clones of HCT116^{p53-/-}PSCA with p53-transposition and mock-transposition, respectively, were pooled and 1×10³ cells thereof were transferred to 100 mm cell culture dishes, grown for additional three weeks and fixed, stained with crystal violet and quantified using ImageJ software. Yet, transgenic p53 still resulted in less clone numbers when compared to mock-treated controls. In an another experiment surviving clones were expanded, treated for 4 h with DNA-damaging zeocin, and total cell lysates were analyzed in Western Blot to demonstrate induction of transgenic p53 and downstream effector molecule p21^{waf/cip} in HCT116^{p53-/-}PSCA with targeted transposition of p53 **(****Fig. 14c****).** In untreated HCT116^{p53-/-}PSCA cell with targeted transposition of p53 and in controls with targeted mock-transposition no stabilization of p53 and no induction of p21^{waf/cip} expression was noted.

### Examples of the invention

Throughout the figures and the examples of the invention, whe reference is made to BAP, BAP means the BAP of SEQ ID NO. 2.

### Example 1

### Synthesis of cyclodextrin-PPIs and mono-biotinylated mal19-PPI-biotin molecules

Synthesis of 4th generation maltose-modified PPI dendrimers (mal19-PPI (G4) 14,900 g/mol) and biotinylated mal19-PPIs (mall9-PPI-biotin (G4), 15,490 g/mol) were described previously (Jugel et al., 2021; Tietze et al., 2017). For synthesis of β-cyclodextrin-modified PPIs **(****Fig. 3****),** the complete reactions were carried out under argon protection atmosphere. In a first reaction flask (heated, degassed and filled with argon), 2nd and 4th generation PPI den-drimers were added and degassed for around 1 h under high vacuum, followed by the addition of 2 mL of degassed anhydrous DMSO and triethylamine (Et₃N). In a second reaction flask, (heated, degassed and filled with argon) βCD-PEG-CO₂H and BOP were dis-solved in 2 ml of degassed anhydrous DMSO. Subsequently, the resulting reaction mixture was stirred for 2 h. PPI dendrimer solution was slowly added to the activated βCD-PEG-CO₂H solution, stirred for 2-2.5 days at room temperature and dialyzed for 2 days in 5 l water (membrane tube with MWCO 2000 g/mol) with exchanging aqueous solution. After the freeze drying a viscous liquid was obtained which for subsequent experiments was reconstituted in distilled water.

### Example 2

### Toxicity of PPIs and modified PPIs of generation G2 and G4

Cell viability of 293T cells incubated with increasing concentrations of different types of PPI molecules was assessed. 2x 10⁴ 293T cells were plated in 96 well plates and grown in supplemented DMEM until 70 % confluency. Different PPI molecules were added to cells at increasing concentrations and viability was tested after 24 h using AlamarBlue Assay (Thermo Fisher Scientific Inc., Waltham, USA). More specifically, 20 µl AlamarBlue solution was added to wells containinig 200µl medium and cells, and plates were incubated for additional 5 h. As positive control cells were lysed with 5 % Triton X-100 (Sigma-Aldrich). Untreated cells were included as negative control. Subsequently, fluorescence intensity of the reduced AlamarBlue was measured using a fluorescence imaging system (Synergy 2TM, BioTek, Winooski, USA) and 560EX nm/590EM nm filter settings. The cytotoxicity of PPI-molecules on cells was normalized to untreated controls, which were set to 100 % viability. **Figure 4** demonstrates negligible cytotoxicity of all PPI derivatives analyzed at concentrations at 2.5 µM and below. The cytotoxicity of PPI dendrimers increased with the PPI generation. PPI (G2) was essentially non-toxic even at a concentration of 10 µM, whereas the LD50 value calculated for PPI (G4) was 3.5 µM. After surface modification of PPIs with β-cyclodextrin, PPI (G2) remained non-toxic, remarkably the LD50 value of PPI (G4) increased up to 4.75 µM, suggesting that shielding of the peripheral primary amino surface groups by β-cyclodextrin decreased cytotoxicity.

### Example 3

### Analysis of dendriplex formation using electrophoretic mobility gel shift and analyses of size and zeta potential of formed polyplexes using multiparameter nanoparticle tracking

Minicircle DNA (1 µg) was incubated for 30 min at room temperature with increasing amounts of PPI or CD-PPI corresponding to mass ratios of 1:5 to 1:0.2. The dendriplexes were then separated by agarose gel electrophoresis [1 % (w/v)] and analyzed under UV light (G:Box Chemi XX9). As depicted in **Fig. 5****,** MC mobility was completely inhibited regardless of surface modification at MC-DNA/PPI mass ratios 1:5, 1:3 and 1:1, indicating that the positive net charge of the dendrimers was sufficient for MC complexation. As shown in **Fig. 10****,** multiparameter nanoparticle tracking analysis was performed using the ZetaView^{®} PMX120 (Particle Metrix GmbH) according to the manufacturer's instructions to determine the zeta potential and the particle size of scFv(AM1)-P-BAP-hybrid polyplexes with MC. Data were analyzed using the manufacturer's software (ZetaView 8.05.05). Average particle size of scFv(AM1)-P-BAP- hybrid polyplexes containing G2 cyclodextrin-PPIs (CD-PPIs) were in the mean 146.5 ± 56.3 nm and accompanied by an unexpected average negative net charge of - 16,8 ± 0.2 mV. Similar data was obtained for hybrid polyplexes containing G4 CD-PPIs (mean diameter, 136.9 ± 55.1 nm; net charge -11.7 ± 0.3 mV). The net charge of the intermediate CD-PPI (G2)/MC and CD-PPI (G4)/MC dendrimers was positive (5.3 ± 0.2 mV, and 3.7 ± 0.1 mV, respectively), This indicates that assembly of hybrid polyplexes by complexation between bioconjugation adduct 1 and bioconjugation adduct 2 is accomplished by electrostatic interactions **(****Fig. 2****)** and in the end result in masking previous positive net charge of the dendriplexes (adduct 2). Yet, mal19-PPI-biotin/neutravidin/scFv-P-BAP polyplexes had a much more negative net charge, up to -30.8 ± 0.5 mV **(****Fig. 10****).**

### Example 5

### Generation biotinylated single chain antibodies

The DNA sequence and features of the single chain antibody-derivative scFv(AM1)-P-BAP have been described previously (Jugel et al., 2021). The construct includes an N-terminal Igκ leader sequence, a biotin acceptor peptide (P-BAP) and a C-terminal c-myc epitope and a 6x histidine (His)-tag. For production of biotinylated scFvs, a 293T^{huBirA} producer cell line containing a codon-optimized biotin ligase was employed (Tietze et al., 2017). After transient transfection of vectors encoding scFv-P-BAP constructs (see Fig 7A) in 293T^{huBirA} cells harvested cell culture supernatant containing biotinylated and His6-tagged scFv-P-BAPs was subjected to Ni²⁺-NTA affinity chromatography. Briefly, 50 ml of clarified supernatant was passed through a Ni²⁺-NTA spin column (Qiagen) and washed with 1X PBS containing 150 mM NaCl and 10 mM/20 mM imidazole. Elution of bound scFvs was performed in 500 µl 1X PBS containing 150 mM NaCl and 350 mM imidazole per column. Eluted scFvs were dialyzed in 1X PBS twice for 2 h and additionally for 12 h at 4 °C. The biotinylated scFvs were further purified using avidin-biotin affinity chromatography with monomeric avidin columns (Thermo Fisher Scientific) according to the protocol of the manufacturer. Eluted scFvs were dialyzed again as described previously. The recombinant proteins were subsequently concentrated using Amicon tubes Ultra-15 (Merck Millipore) and were stored in aliquots at - 20 °C until use. scFv-P-BAPs were subjected to SDS-poylacrylamide-gelectrophoresis, blotted onto nitrocellulose filters and probed with anti-c-myc and anti-biotin antibodies **(****Fig. 8****)** to demonstrate full length scFv-P-BAPs and the biotin residue. In parallel, purity of the recombinant proteins were assessed in Commassie Brilliant Blue-stained SDS-PAGE gels **(****Fig. 8****).** Binding of scFv(AM1)-P-BAP to cognate PSCA receptor and accessibility of the biotin residue of the single-chain antibody was confirmed in FACS analysis using 293T, HCT116^{p53-/-}, H4 wild-type cells and isogenic cell lines thereof genetically engineered to express PSCA (293T-PSCA, HCT116^{p53-/-}-PSCA, H4-PSCA) (Fig. 9). As additional control a scFv-P-BAP specific for a neo-epitope in the epidermal growth factor receptor variant III (EGFRvIII) was used.

### Example 6

### Vector constructs, production of minicircle (MC) DNAs and assembly of tumor-specific hybrid polyplexes for targeted transfection

To generate a minicircle transposon encoding GFP, the synthetic transposase restriction sites IR/DR(L) and IR/DR(R) were ligated to the corresponding restriction sites Smal - Clal and StuI- EcoRV of the parental minicircle vector pMC.CMV-GFP (System Biosciences). A synthetic codon-optimized cDNA encoding the full 393 amino acids of p53 (Eurofins MWG Biotech) fused to a T2A endoproteolytic cleavage site and a puromycin resistance gene was ligated to the corresponding restriction sites Clal and HindIII of the parental minicircle vector pMC.CMV (System Biosciences), resulting in pMC-p53-puroR. In pMC-p53-puroR, the p53/puroR transgene was flanked by transposase restriction sites IR/DR(L) and IR/DR(R). pMC-puroR lacking the p53 coding sequence was used as mock control. The pCMV(CAT)T7-SB100 (Addgene, plasmid # 34879) encoding hyperactive SB100X Sleeping Beauty Transposase has been described previously (Mates et al., 2009). The SB100X gene sequence was amplified by PCR adding Xbal restriction sites and ligated to the Xbal restriction sites in the MCS of the parental minicircle vector pMC.CMV-MCS (System Biosciences). All vector inserts were confirmed by sequencing (Microsynth Seqlab).

MC vectors were produced using the MC Easy^{™} Minicircle DNA Production Kit (System Biosciences) according to the manufacturer's protocol. Briefly, pMC-GFP, pMC-puroR, pMC-p53-puroR and pMC-SB100 were grown in E. coli bacterial strain ZYCY10P3S2T harboring an arabinose-inducible system for simultaneous expression of PhiC31 integrase and See I endonuclease. After incubation with induction medium, intramolecular (cis-) recombination generated MC from the parental plasmid mediated by PhiC31 integrase. The remaining parental plasmid-DNA backbone was degraded by Sce-I endonuclease. MC-GFP (3738 bp), MC-puroR (2.6 kp), MC-p53-puroR (3.7 kp) and MC-SB100 (4.5 kp) were purified from medium using Plasmid Plus Maxi Kit (Qiagen) according to the manufacturer's protocol.

For the assembly of tumor-specific hybrid polyplexes a two-step conjugation protocol was used as depicted in **Fig. 2****.** First, CD-PPIs were mixed with MC at a mass ratio of 5:1, resulting in bioconjugation adduct 1. In parallel, neutravidin (Thermo Fisher Scientific), scFv-P-BAP and ma119-PPI-biotin were incubated for 30 min at room temperature at a molar ratio of 2:1:1, resulting in bioconjugation adduct 2. After saturation of the remaining free biotin binding sites of neutravidin with 0.3 mM D-biotin (Sigma Aldrich), the intermediate conjugates were mixed for 30 min at room temperature and subsequently used further studies. Hybrid polyplexes with 1 µg MC-DNA contain 100 pmol scFv-P-BAP, 50 pmol neutravidin and 50 pmol mal19-PPI-biotin. 24h before transfection with polyplexes, indicated amounts of target cells were plated in medium containing heat-inactivated FCS and treated with 6.5 - 7.5×10³ hybrid polyplexes /cell for 12 hours before exchanging with fresh medium.

### Example 7

### Assessment of particle size and zeta potential of polyplexes

Multiparameter nanoparticle tracking analysis of polyplexes was performed using the ZetaView^{®} PMX120 (Particle Metrix GmbH) according to the manufacturer's instructions to determine the zeta potential and the particle size of scFv(AM1)-P-BAP-hybrid polyplexes with MC. Data were analyzed using the manufacturer's software (ZetaView 8.05.05). As depicted in **Fig. 10** assessment of the physiochemical properties of scFv-P-BAP conjugated neutravidin, dendriplexes and polyplexes revealed a negative net charge for the intermediate conjugate mal19-PPI-biotin/neutravidin/scFv-P-BAP (molar ratio 1:1:2) (-30.8 ± 0.5 mV) and a positive net charge for CD-PPI dendriplexes (G2) of 5.3 ± 0.2 mV and for CD-PPI dendriplexes (G4) 5.3 ± 0.2 mV, 3.7 ± 0 mV (mass ratio of CD-PPI to DNA at 5:1). Complexation between both intermediate conjugates resulted in a mean negative net charge of -16.8 ± 0.2 mV and -11.7 ± 0.3 mV and a mean particle size of 146.5 ± 72.9 nm and 136.9 ± 55.1 nm for scFv(AM1)-guided hybrid polyplexes containing CD-PPI (G2) or (G4), respectively.

### Example 8

### Receptor-mediated endocytosis of PSCA-specific hybrid polyplexes

Binding of scFv(AM1)-P-BAP-hybrid polyplexes loaded with Cy3-labeled DNA to PSCA-positive target cells was analyzed by flow cytometry. As negative control, non-specific hybrid polyplexes loaded with Cy3-labeled DNA and containing the EGFRvIII-specific scFv(MR1.1)-P-BAP were included. 2×10⁵ 293T-PSCA cells were treated with the different hybrid polyplexes for 4h. After Heparin-washing of surface-bound antibodies, the internalized Cy3-labelled plasmid DNA was measured by flow cytometry. Non-treated 293T.PSCA cells were included in the FACS analyses.

As depicted in **Fig. 11A****, B** flow cytometry analysis demonstrated scFv(AM1)-P-BAP-mediated internalization of Cy3-labeled DNA by 293TPSCA cells, whereas significantly less Cy3 signals are detected in cells after treatment with non-specific hybrid polyplexes conjugated with scFv(MR1.1)-P-BAP.

Confocal laser scanning microscopy studies, shown in Figure **4C+D**, supported the data obtained by flow cytometry analysis. The tumor-specific scFv(AM1)-P-BAP hybrid polyplexes were found to be internalized by 293T-PSCA cells. Staining with a wheat germ agglutinin conjugate revealed that the Cy3-labelled plasmid DNA was located intracellularly in the cytoplasm. Additional staining with the early endosome associated protein EEA1 (early endosome antigen 1) demonstrated partial location in early endosomes. In contrast, the non-specific scFv(MR1.1)-P-BAP-guided hybrid polyplexes showed essentially less nanoparticle uptake. Quantitative analysis of Cy3 signals revealed significantly more signals overall and per cell after treatment with scFv(AM1)-P-BAP hybrid polyplexes compared to control hybrid polyplexes (**Figure 4E+F**).

To investigate if a receptor-mediated endocytosis contributes to uptake of scFv-P-BAP hybrid polyplexes clathrin-dependent and clathrin-independent endocytosis was blocked using chlorpromazine and filipin III. Both, filipin III and chlorpromazine treatment of cells lead to significant decrease in the fraction of GFP-positive 293T-PSCA cells when transfected with scFv(AM1)-P-BAP hybrid polyplexes loaded with MC encoding GFP. None of the used endocytosis inhibitors completely blocked transfection of GFP suggesting that besides clathrin-dependent and -independent endocytic pathways a direct uptake mechanisms of scFv-P-BAP-guided hybrid polyplexes might have supported delivery of MCs encoding GFP..

### Example 9

### Targeted delivery of p53 transposon to PSCA-positive target cells

Minicircle DNA containing a transposon encoding p53 and puromycin resistance (*pac* gene) and minicircle DNA encoding hyperactive Sleeping Beauty transposase SB100X were assembled in hybrid polyplexes targeting PSCA. As control PSCA-specific hybrid polyplexes containing minicircle DNA encoding a transposon encoding puromycin resistance and minicircle encoding SB100X were employed. For analysis of direct effects after targeted p53 transposition, 2×10⁴ HCT116^{p53-/-/PSCA} or H4^{PSCA} cells plated in 6-well plates in DMEM complete were transfected with hybrid polyplexes. Treated cells were continuously grown in medium containing 2 µg/ml puromycin starting 24 h after transfection. Clonogenic survival was analyzed after 14 days by staining fixed cell clones with crystal violet staining solution (Merck KGaA). As depicted in **Fig. 14A****,** targeted transposition of TP53 revealed decreased clonogenic survival of HCT116^{p51-/-/PSCA} transfected with p53-transposon when compared to mock controls. The effect of targeted p53 transposition was even more increased in transfected H4-PSCA glioma cells when compared to the control **(****Fig. 14A****).** Surviving H4^{PSCA} glioma cell clones had in general fewer cell numbers (data not shown) and pooled clones could not be further propagated. Pooled HCT116^{p53-/-/PSCA} cell clones that survived scFv(AM1)-P-BAP-guided transposition of p53 were readily propagated onto new culture dishes but showed less clonal survival than cells transfected with hybrid polyplexes delivering a minicircle transposon for puromycin resistance and minicircle for SB100X . **(****Fig. 14B****).** Western blot analysis of cell lysates from HCT116^{p53-/-/PSCA} cells with stable transgenic p53 transposition showed after treatment with DNA-damaging antibiotic zeocin a posttranslational p53 phosphorylation and stabilization leading to induced expression of p53's transcriptional target p21waf/cip **(****Fig 14C****)**

### References

Al-Dosari, M.S., Gao, X., 2009. Nonviral gene delivery: principle, limitations, and recent progress. AAPS J. 11, 671-681.

Alessio, N., Aprile, D., Cappabianca, S., Peluso, G., Di Bernardo, G., Galderisi, U., 2021. Different Stages of Quiescence, Senescence, and Cell Stress Identified by Molecular Algorithm Based on the Expression of Ki67, RPS6, and Beta-Galactosidase Activity. Int. J. Mol. Sci. 22, 3102.

Bäumer, N., Appel, N., Terheyden, L., Buchholz, F., Rossig, C., Müller-Tidow, C., Berdel, W.E., Bäumer, S., 2016. Antibody-coupled siRNA as an efficient method for in vivo mRNA knockdown. Nat. Protoc. 11, 22-36.

Check, E., 2002. A tragic setback. Nature.

Dinauer, N., Lochmann, D., Demirhan, I., Bouazzaoui, A., Zimmer, A., Chandra, A., Kreuter, J., von Briesen, H., 2004. Intracellular tracking of protamine/antisense oligonucleotide nanoparticles and their inhibitory effect on HIV-1 transactivation. J. Control. Release 96, 497-507.

Dufès, C., Uchegbu, I.F., Schätzlein, A.G., 2005. Dendrimers in gene delivery. Adv. Drug Deliv. Rev. 57, 2177-2202.

Hayashi, Y., Takamiya, M., Jensen, P.B., Ojea-Jiménez, I., Claude, H., Antony, C., Kjaer-Sorensen, K., Grabher, C., Boesen, T., Gilliland, D., Oxvig, C., Strähle, U., Weiss, C., 2020. Differential Nanoparticle Sequestration by Macrophages and Scavenger Endothelial Cells Visualized in Vivo in Real-Time and at Ultrastructural Resolution. ACS Nano 14, 1665-1681.

Hui, S.-W., 2008. Overview of drug delivery and alternative methods to electroporation. Methods Mol. Biol. 423, 91-107.

Jugel, W., Aigner, A., Michen, S., Hagstotz, A., Ewe, A., Appelhans, D., Schackert, G., Temme, A., Tietze, S., 2021. Targeted RNAi of BIRC5/Survivin Using Antibody-Conjugated Poly(Propylene Imine)-Based Polyplexes Inhibits Growth of PSCA-Positive Tumors. Pharm..

Lee, J.C., Bermudez, H., Discher, B.M., Sheehan, M.A., Won, Y.Y., Bates, F.S., Discher, D.E., 2001. Preparation, stability, and in vitro performance of vesicles made with diblock copolymers. Biotechnol. Bioeng. 73, 135-145.

Li, X., Stuckert, P., Bosch, I., Marks, J.D., Marasco, W.A., 2001. Single-chain antibody-mediated gene delivery into ErbB2-positive human breast cancer cells. Cancer Gene Ther. 8, 555-565.

Mates, L., Chuah, M.K.L., Belay, E., Jerchow, B., Manoj, N., Acosta-Sanchez, A., Grzela, D.P., Schmitt, A., Becker, K., Matrai, J., Ma, L., Samara-Kuko, E., Gysemans, C., Pryputniewicz, D., Miskey, C., Fletcher, B., VandenDriessche, T., Ivics, Z., Izsvák, Z., 2009. Molecular evolution of a novel hyperactive Sleeping Beauty transposase enables robust stable gene transfer in vertebrates. Nat. Genet. 41, 753-761.

Morgenroth, A., Cartellieri, M., Schmitz, M., Günes, S., Weigle, B., Bachmann, M., Abken, H., Rieber, E.P., Temme, A., 2007. Targeting of tumor cells expressing the prostate stem cell antigen (PSCA) using genetically engineered T-cells. Prostate 67, 1121-1131.

Pardridge, W.M., Buciak, J.L., Kang, Y.S., Boado, R.J., 1993. Protamine-mediated transport of albumin into brain and other organs of the rat. Binding and endocytosis of protamine-albumin complex by microvascular endothelium. J. Clin. Invest. 92, 2224-2229.

Plückthun, A., 1994. Antibodies from Escherichia coli BT - The Pharmacology of Monoclonal Antibodies, in: Rosenberg, M., Moore, G.P. (Eds.), . Springer Berlin Heidelberg, Berlin, Heidelberg, pp. 269-315.

Shcharbin, D., Pedziwiatr, E., Bryszewska, M., 2009. How to study dendriplexes I: Characterization. J. Control. Release 135, 186-197.

Smith, T.T., Stephan, S.B., Moffett, H.F., McKnight, L.E., Ji, W., Reiman, D., Bonagofski, E., Wohlfahrt, M.E., Pillai, S.P.S., Stephan, M.T., 2017. In situ programming of leukaemia-specific T cells using synthetic DNA nanocarriers. Nat. Nanotechnol. 12, 813-820.

Suzuki, M., Takayanagi, A., Shimizu, N., 2003. Recombinant single-chain antibodies with various oligopeptide tails for targeted gene delivery. Gene Ther. 10, 781-788.

Tan, P.H., Manunta, M., Ardjomand, N., Xue, S.A., Larkin, D.F.P., Haskard, D.O., Taylor, K.M., George, A.J.T., 2003. Antibody targeted gene transfer to endothelium. J. Gene Med. 5, 311-323.

Theoharis, S., Krueger, U., Tan, P.H., Haskard, D.O., Weber, M., George, A.J.T., 2009. Targeting gene delivery to activated vascular endothelium using anti E/P-Selectin antibody linked to PAMAM dendrimers. J. Immunol. Methods 343, 79-90.

Thomas, C.E., Ehrhardt, A., Kay, M.A., 2003. Progress and problems with the use of viral vectors for gene therapy. Nat. Rev. Genet. 4, 346-358.

Tietze, S., Schau, I., Michen, S., Ennen, F., Janke, A., Schackert, G., Aigner, A., Appelhans, D., Temme, A., 2017. A Poly(Propyleneimine) Dendrimer-Based Polyplex-System for Single-Chain Antibody-Mediated Targeted Delivery and Cellular Uptake of SiRNA. Small 13.

## Claims

1. A delivery system for targeted delivery of a therapeutically active payload, comprising
• an avidin core, wherein the avidin core consists of a avidin, neutravidin or streptavidin molecule;
• at least one targeting molecule selected from the group consisting of antibodies, antibody single-chain variable fragments (scFv) and aptamers,
• at least one nucleic acid payload selected from DNA and RNA,
wherein said nucleic acid payload is complexed with a biotinylated hybrid polyplex comprising maltose-PPI dendrimers and cyclodextrin-PPI dendrimers, wherein PPI is defined as poly(propylene imine) (PPI); and wherein said at least one targeting molecule and said biotinylated hybrid polyplex are conjugated to said avidin core.

2. The delivery system according to claim 1, comprising
a. one antibody single-chain variable fragment and three therapeutically active nucleic acids, or
b. two antibody single-chain variable fragments and two therapeutically active nucleic acids, or
c. three antibody single-chain variable fragments and one therapeutically active nucleic acid.

3. The delivery system according to claim 1 or 2, wherein said antibody single-chain variable fragment is linked to a biotinylation acceptor peptide (BAP) either directly or via a linker peptide.

4. The delivery system according to claim 3, wherein said antibody single-chain variable fragment is comprised in a construct, having the structure:
antibody single-chain variable fragment - BAP; or
antibody single-chain variable fragment - linker - BAP;
and wherein this construct is mono-biotinylated at the BAP.

5. The delivery system according to any one of claims 1 to 4, wherein the antibody single-chain variable fragment is selected from scFv(AM1) (SEQ ID NO: 1), scFv(h-AM-1) (SEQ ID NO: 12) and scFv(MR1.1) (SEQ ID NO: 13).

6. The delivery system according to any one of claims 3 to 5, wherein said BAP is selected from the group consisting of:
• 
MKLKVTVNGTAYDVDVDVDKSHENPMGTILFGGGTGGAPAPAAGGA
GAGKAGEGEIPAPLAGTVSKILVKEGDTVKAGQTVLVLEAMKMETEIN
APTDGKVEKVLVKERDAVQGGQGLIKIGDLEL (SEQ ID NO. 2);
• 
VLRSPMPGVVVAVSVKPGDAVAEGQEICVIEAMKMQNSMTAGKTGT
VKSVHCQAGDTVGEGDLLVELE (SEQ ID NO: 3);
∘ LX₁X₂IFEAQKIEWR (SEQ ID NO: 4), wherein
X₁ = any amino acid; and
X₂ = is any amino acid except L, V, I, W, F or Y;
∘ GLNDIFEAQKIEWHE (SEQ ID NO. 5);
∘ ALNDIFEAQKIEWHA (SEQ ID NO: 6);
∘ MAGGLNDIFEAQKIEWHEDTGGS (SEQ ID NO. 7);
MSGLNDIFEAQKIEWHEGAPSSR (SEQ ID NO: 8); and
∘ LHHILDAQKMVWNHR (SEQ ID NO: 9);
or wherein said linker peptide is selected from the group consisting of
• two amino acids, such as GS;
• 6 amino acids; and
• 10 amino acids, such as the c-myc tag having the amino acid sequence of EQKLISEEDL (SEQ ID NO: 10).

7. The delivery system according to any one of claims 1 to 6, wherein the maltose-PPI dendrimers in said hybrid polyplexes are mal19-PPI dendrimers.

8. The delivery system according to any one of claims 1 to 6, wherein the cyclodextrin-PPI dendrimers in said hybrid polyplexes are β-cyclodextrin-modified PPI dendrimers of the second generation (G2) modified comprising 31 % β-cyclodextrin and of the fourth generation (G4) comprising 7 % β-cyclodextrin.

9. The delivery system according to any one of claims 1 to 8, wherein said therapeutically active payload is a therapeutically active nucleic acid selected from the group consisting of CpG oligonucleotides, ssDNA, dsDNA, ssRNA, dsRNA, plasmid DNA and minicircle DNA, preferably plasmid DNA and minicircle DNA.

10. The delivery system according to any one of the preceding claims, wherein said therapeutically active payload is plasmid DNA and minicircle DNA, wherein said plasmid DNA or minicircle-DNA encodes the human tumor suppressor gene *p53* (SEQ ID NO: 11).

11. The delivery system according to any one of the preceding claims, comprising
• a neutravidin molecule as the avidin core;
• at least one scFv(AM1) (SEQ ID NO: 1) antibody single-chain variable fragment (scFv) - linker peptide - BAP as the targeting molecule, which is biotinylated; wherein
said BAP is 
MKLKVTVNGTAYDVDVDVDKSHENPMGTILFGGGTGGAPAPAAG
GAGAGKAGEGEIPAPLAGTVSKILVKEGDTVKAGQTVLVLEAMKM
ETEINAPTDGKVEKVLVKERDAVQGGQGLIKIGDLELEL (SEQ ID NO.
 2); and
wherein said linker peptide is c-myc tag having the amino acid sequence of EQKLISEEDL (SEQ ID NO: 10);
• at least one therapeutically active nucleic acid, which is selected from at least one plasmid DNA molecule and at least one minicircle DNA molecule, wherein said at least one plasmid DNA molecule or said at least one minicircle DNA molecule is preferably encoding p53 (SEQ ID NO. 11);
wherein said at least one therapeutically active nucleic acid is complexed with a biotinylated hybrid polyplex comprising maltose-PPI and cyclodextrin-PPI, wherein PPI is defined as poly(propylene imine) (PPI) and wherein said at least one targeting molecule and said biotinylated hybrid polyplex are conjugated to said avidin core.

12. A process for the assembly of a delivery system according any one of the preceding claims comprising the steps of:
a) preparing conjugates comprising biotinylated targeting molecules conjugated to avidin, neutravidin or streptavidin,
b) preparing cyclodextrin-PPI dendrimers,
c) preparing maltose-PPI-biotin dendrimers;
d) incubating maltose-PPI-biotin dendrimers with the conjugates resulting from step a), wherein the maltose-PPI has a cationic charge;
e) binding the maltose-PPI-biotin dendrimers to the conjugates resulting from step a);
f) separately, incubating cyclodextrin-PPI with nucleic acid, wherein cyclodextrin-PPI-nucleic acid dendrimers are formed, which have a weak anionic charge,
g) incubating the complexes resulting from step e) with the cyclodextrin-PPI-nucleic acid dendrimers of step f), and
h) formation, through ionic interaction, of a tumor-targeting delivery system, comprising biotinylated targeting molecules and hybrid polyplexes comprising maltose-PPI-biotin/cyclodextrin-PPI dendrimers which contain the nucleic acid payload, wherein said biotinylated targeting molecules and said hybrid polyplexes are bound to the avidin/neutravidin core.

13. The process according to claim 12 comprising the steps of
a) preparing conjugates comprising biotinylated scFv-linker peptide-BAP conjugated to avidin, neutravidin or streptavidin;
b) preparing cyclodextrin-PPI dendrimers,
c) preparing maltose-PPI-biotin dendrimers;
d) incubating maltose-PPI-biotin dendrimers with the conjugates resulting from step a), wherein the maltose-PPI has a cationic charge;
e) binding the maltose-PPI-biotin dendrimers to the conjugates resulting from step a);
f) separately, incubating cyclodextrin-PPI dendrimers with a nucleic acid, wherein cyclodextrin-PPI-nucleic acid dendrimers are formed, which have a weak anionic charge,
g) incubating the complexes resulting from step e) with the cyclodextrin-PPI-nucleic acid dendrimers of step f), and
h) formation, through ionic interaction, of a tumor-targeting delivery system, comprising biotinylated scFv-linker peptide-BAP and hybrid polyplexes comprising maltose-PPI-biotin/cyclodextrin-PPI dendrimers which contain the nucleic acid payload, wherein said biotinylated scFv-linker peptide-BAP and said hybrid polyplexes are bound to the avidin/neutravidin core.

14. A pharmaceutical composition comprising the delivery system according to any one of claims 1-11, wherein said pharmaceutical composition optionally further comprises other anti-tumor drugs such as nucleic agonists for endosomal and cytosolic pattern recognition receptors selected from TLR3, TLR7, TLR9, RIG-I, and MDA5; and/or EGF receptor inhibitor selected from
• tyrosine kinase inhibitors or monoclonal antibodies;
• gefitinib, erlotinib, afatinib and osimertinib and cetuximab; and
• CimaVax-EGF.

15. The delivery system according to any one of claims 1-11 or a pharmaceutical composition comprising the delivery system according to any one of claims 1-11 for use in the treatment of metabolic diseases, such as familial hypercholesterolemia, viral infections, and proliferative diseases, such as primary tumors like glioblastoma multiforme (GBM), pancreatic cancer, prostate cancer, urogenital tumors or metastatic cancers.

## Amended claims

### Amended claims in accordance with Rule 137(2) EPC.

1. A delivery system for targeted delivery of a therapeutically active payload, comprising
• an avidin core, wherein the avidin core consists of a avidin, neutravidin or streptavidin molecule;
• at least one biotinylated targeting molecule selected from the group consisting of antibodies, antibody single-chain variable fragments (scFv) and aptamers,
• at least one nucleic acid payload selected from DNA and RNA,
wherein said nucleic acid payload is complexed with a biotinylated hybrid polyplex comprising maltose-PPI dendrimers and cyclodextrin-PPI dendrimers, wherein PPI is defined as polypropylene imine) (PPI); and wherein said at least one targeting molecule and said biotinylated hybrid polyplex are conjugated to said avidin core.

2. The delivery system according to claim 1, comprising
a. one antibody single-chain variable fragment and three therapeutically active nucleic acids, or
b. two antibody single-chain variable fragments and two therapeutically active nucleic acids, or
c. three antibody single-chain variable fragments and one therapeutically active nucleic acid.

3. The delivery system according to claim 1 or 2, wherein said antibody single-chain variable fragment is linked to a biotinylation acceptor peptide (BAP) either directly or via a linker peptide.

4. The delivery system according to claim 3, wherein said antibody single-chain variable fragment is comprised in a construct, having the structure:
antibody single-chain variable fragment - BAP; or
antibody single-chain variable fragment - linker - BAP;
and wherein this construct is mono-biotinylated at the BAP.

5. The delivery system according to any one of claims 1 to 4, wherein the antibody single-chain variable fragment is selected from scFv(AM1) (SEQ ID NO: 1), scFv(h-AM-1) (SEQ ID NO: 12) and scFv(MR1.1) (SEQ ID NO: 13).

6. The delivery system according to any one of claims 3 to 5, wherein said BAP is selected from the group consisting of:
• 
MKLKVTVNGTAYDVDVDVDKSHENPMGTILFGGGTGGAPAPAAGGA
GAGKAGEGEIPAPLAGTVSKILVKEGDTVKAGQTVLVLEAMKMETEIN
APTDGKVEKVLVKERDAVQGGQGLIKIGDLEL (SEQ ID NO. 2);
• 
VLRSPMPGVVVAVSVKPGDAVAEGQEICVIEAMKMQNSMTAGKTGT
VKSVHCQAGDTVGEGDLLVELE (SEQ ID NO: 3);
∘ LX₁X₂IFEAQKIEWR (SEQ ID NO: 4), wherein
X₁ = any amino acid; and
X₂ = is any amino acid except L, V, I, W, F or Y;
o GLNDIFEAQKIEWHE (SEQ ID NO. 5);
o ALNDIFEAQKIEWHA (SEQ ID NO: 6);
o MAGGLNDIFEAQKIEWHEDTGGS (SEQ ID NO. 7);
MSGLNDIFEAQKIEWHEGAPSSR (SEQ ID NO: 8); and
o LHHILDAQKMVWNHR (SEQ ID NO: 9);
or wherein said linker peptide is selected from the group consisting of
• two amino acids, such as GS;
• 6 amino acids; and
• 10 amino acids, such as the c-myc tag having the amino acid sequence of EQKLISEEDL (SEQ ID NO: 10).

7. The delivery system according to any one of claims 1 to 6, wherein the maltose-PPI dendrimers in said hybrid polyplexes are mal 19-PPI dendrimers.

8. The delivery system according to any one of claims 1 to 6, wherein the cyclodextrin-PPI dendrimers in said hybrid polyplexes are β-cyclodextrin-modified PPI dendrimers of the second generation (G2) modified comprising 31 % β-cyclodextrin and of the fourth generation (G4) comprising 7 % β-cyclodextrin.

9. The delivery system according to any one of claims 1 to 8, wherein said therapeutically active payload is a therapeutically active nucleic acid selected from the group consisting of CpG oligonucleotides, ssDNA, dsDNA, ssRNA, dsRNA, plasmid DNA and minicircle DNA, preferably plasmid DNA and minicircle DNA.

10. The delivery system according to any one of the preceding claims, wherein said therapeutically active payload is plasmid DNA and minicircle DNA, wherein said plasmid DNA or minicircle-DNA encodes the human tumor suppressor gene *p53* (SEQ ID NO: 11).

11. The delivery system according to any one of the preceding claims, comprising
• a neutravidin molecule as the avidin core;
• at least one scFv(AM1) (SEQ ID NO: 1) antibody single-chain variable fragment (scFv) - linker peptide - BAP as the targeting molecule, which is biotinylated; wherein
said BAP is 
MKLKVTVNGTAYDVDVDVDKSHENPMGTILFGGGTGGAPAPAAG
GAGAGKAGEGEIPAPLAGTVSKILVKEGDTVKAGQTVLVLEAMKM
ETEINAPTDGKVEKVLVKERDAVQGGQGLIKIGDLEL (SEQ ID NO.
2); and
 wherein said linker peptide is c-myc tag having the amino acid sequence of EQKLISEEDL (SEQ ID NO: 10);
• at least one therapeutically active nucleic acid, which is selected from at least one plasmid DNA molecule and at least one minicircle DNA molecule, wherein said at least one plasmid DNA molecule or said at least one minicircle DNA molecule is preferably encoding p53 (SEQ ID NO. 11);
wherein said at least one therapeutically active nucleic acid is complexed with a biotinylated hybrid polyplex comprising maltose-PPI and cyclodextrin-PPI, wherein PPI is defined as polypropylene imine) (PPI) and wherein said at least one targeting molecule and said biotinylated hybrid polyplex are conjugated to said avidin core.

12. A process for the assembly of a delivery system according any one of the preceding claims comprising the steps of:
a) preparing conjugates comprising biotinylated targeting molecules conjugated to avidin, neutravidin or streptavidin,
b) preparing cyclodextrin-PPI dendrimers,
c) preparing maltose-PPI-biotin dendrimers;
d) incubating maltose-PPI-biotin dendrimers with the conjugates resulting from step a), wherein the maltose-PPI has a cationic charge;
e) binding the maltose-PPI-biotin dendrimers to the conjugates resulting from step a);
f) separately, incubating cyclodextrin-PPI with nucleic acid, wherein cyclodextrin-PPI-nucleic acid dendrimers are formed, which have a weak anionic charge,
g) incubating the complexes resulting from step e) with the cyclodextrin-PPI-nucleic acid dendrimers of step f), and
h) formation, through ionic interaction, of a tumor-targeting delivery system, comprising biotinylated targeting molecules and hybrid polyplexes comprising maltose-PPI-biotin/cyclodextrin-PPI dendrimers which contain the nucleic acid payload, wherein said biotinylated targeting molecules and said hybrid polyplexes are bound to the avidin/neutravidin core.

13. The process according to claim 12 comprising the steps of
a) preparing conjugates comprising biotinylated scFv-linker peptide-BAP conjugated to avidin, neutravidin or streptavidin;
b) preparing cyclodextrin-PPI dendrimers,
c) preparing maltose-PPI-biotin dendrimers;
d) incubating maltose-PPI-biotin dendrimers with the conjugates resulting from step a), wherein the maltose-PPI has a cationic charge;
e) binding the maltose-PPI-biotin dendrimers to the conjugates resulting from step a);
f) separately, incubating cyclodextrin-PPI dendrimers with a nucleic acid, wherein cyclodextrin-PPI-nucleic acid dendrimers are formed, which have a weak anionic charge,
g) incubating the complexes resulting from step e) with the cyclodextrin-PPI-nucleic acid dendrimers of step f), and
h) formation, through ionic interaction, of a tumor-targeting delivery system, comprising biotinylated scFv-linker peptide-BAP and hybrid polyplexes comprising maltose-PPI-biotin/cyclodextrin-PPI dendrimers which contain the nucleic acid payload, wherein said biotinylated scFv-linker peptide-BAP and said hybrid polyplexes are bound to the avidin/neutravidin core.

14. A pharmaceutical composition comprising the delivery system according to any one of claims 1-11, wherein said pharmaceutical composition optionally further comprises other anti-tumor drugs such as nucleic agonists for endosomal and cytosolic pattern recognition receptors selected from TLR3, TLR7, TLR9, RIG-I, and MDA5; and/or EGF receptor inhibitor selected from
• tyrosine kinase inhibitors or monoclonal antibodies;
• gefitinib, erlotinib, afatinib and osimertinib and cetuximab; and
• CimaVax-EGF.

15. The delivery system according to any one of claims 1-11 or a pharmaceutical composition comprising the delivery system according to any one of claims 1-11 for use in the treatment of metabolic diseases, such as familial hypercholesterolemia, viral infections, and proliferative diseases, such as primary tumors like glioblastoma multiforme (GBM), pancreatic cancer, prostate cancer, urogenital tumors or metastatic cancers.
